# EUROPÄISCHE PATENTSCHRIFT

(11) **EP 1 532 950 B1**
(45) Veröffentlichungstag und Bekanntmachung des Hinweises auf die Patenterteilung: **26.03.2008**
(21) Anmeldenummer: 04027322.9
(22) Anmeldetag: 17.11.2004
(51) Int. Cl.: A61F 2/44

(54) **Bandscheibenimplantat**
Spinal disc prosthesis
Prothèse de disque intervertébral

(30) Priorität: 18.11.2003 EP 03026582; 18.05.2004 DE 102004024662
(43) Veröffentlichungstag der Anmeldung: 25.05.2005
(60) Teilanmeldung: 07008698.8
(73) Patentinhaber: Zimmer GmbH, 8404 Winterthur (CH)
(72) Erfinder: Filippi, Michael, 8200 Schaffhausen (CH); Heller, Mathias, 8352 Räterschen (CH); Seebeck, Jörn, 8405 Winterthur (CH); Casutt, Guido, 8544 Rickenbach (CH)
(74) Vertreter: Manitz, Finsterwald & Partner GbR

(56) Entgegenhaltungen:
- EP-A- 0 560 140
- EP-A- 1 405 615
- WO-A-20/04073561
- WO-A-20/05072660
- FR-A- 2 787 017
- US-A1- 2003 135 277
- US-A1- 2003 204 261

## Beschreibung

Die Erfindung betrifft ein Bandscheibenimplantat gemäß Oberbegriff des Anspruchs 1 sowie ein Verfahren zur Herstellung eines Bandscheibenimplantats mit den Merkmalen des Anspruchs 38.

Ein Implantat gemäß Oberbegriff des Anspruchs 1 ist aus EP 1 405 615 A1 bekannt. Ein aus WO 2005/072660 A1 (Stand der Technik gemäß Art. 54(3) EPÜ) bekanntes Implantat unterscheidet sich von dem in Anspruch 1 angegebenen Implantat dadurch, dass einen Implantatkern umgebende Artikulationslagen nicht radial über den Implantatkern hinaus vorstehen.

Künstliche Bandscheiben müssen eine Vielzahl von Anforderungen erfüllen und dabei nicht nur dem Verhalten einer natürlichen Bandscheibe möglichst nahe kommen, sondern beispielsweise auch auf möglichst einfache Weise einsetzbar, d.h. zwischen die jeweiligen beiden benachbarten Wirbelkörper einbringbar sein, sowie hinsichtlich der verwendeten Materialien eine gute Körperverträglichkeit besitzen. Insbesondere die Nachbildung eines möglichst natürlichen elastischen oder dynamischen Verhaltens bei unterschiedlichen Druckverhältnissen, die bei den normalen, auch extreme Belastungen mit sich bringenden Bewegungen der Wirbelsäule auftreten, erweist sich bei der Konzeption von Bandscheibenimplantaten als schwierig.

Aufgabe der Erfindung ist es, ein Bandscheibenimplantat zu schaffen, das allen wesentlichen Anforderungen möglichst gut gerecht wird und insbesondere hinsichtlich des elastischen bzw. dynamischen Verhaltens einer natürlichen Bandscheibe möglichst nahe kommt.

Die Lösung dieser Aufgabe erfolgt durch die Merkmale des Anspruchs 1.

Ein derartiges Bandscheibenimplantat bietet eine Vielzahl von Möglichkeiten, beispielsweise durch Formgebung/ oder Materialwahl das dynamische bzw. elastische Verhalten in der jeweils gewünschten Weise zu beeinflussen. Ferner erweist sich das erfindungsgemäße Bandscheibenimplantat hinsichtlich des Einbringens zwischen zwei benachbarte Wirbelkörper als besonders vorteilhaft. Diesbezüglich wird auf die europäische Patentanmeldung EP 1 532 948 verwiesen, deren Priorität für die vorliegende Anmeldung in Anspruch genommen wird. Diese Prioritätsanmeldung betrifft u.a. ein Operationssystem zum Einsetzen von Bandscheibenimplantaten. Dieses Operationssystem bzw. die Operation selbst sind jedoch nicht Gegenstand der vorliegenden Anmeldung, so dass hierauf nicht näher eingegangen werden soll.

Bevorzugte Ausführungsformen der Erfindung sind auch den abhängigen Ansprüchen, der Beschreibung sowie der Zeichnung zu entnehmen.

Der Implantatkern weist vorzugsweise eine linsenartige Grundform auf. Insbesondere kann der Implantatkern zumindest näherungsweise die Form zweier mit ihren ebenen Seiten aufeinander liegender Kugelsegmente aufweisen, wobei jeweils der Kugelmittelpunkt des einen Kugelsegmentes innerhalb des anderen Kugelsegmentes liegt. Alternativ kann vorgesehen sein, dass der Implantatkern zumindest näherungsweise die Form zweier mit ihren ebenen Seiten einander zugewandter Kugelsegmente und dazwischen liegender Zylinderscheibe aufweist, wobei - wie in der vorstehend genannten Alternative - der Kugelmittelpunkt des einen Kugelsegments innerhalb des anderen Kugelsegments liegt.

Untersuchungen unter Zuhilfenahme von Modellrechnungen haben überraschenderweise ergeben, dass insbesondere unter Beibehaltung der Rotationssymmetrie lokale Belastungsspitzen des Implantatkerns vermieden werden können, wenn spezielle Abstimmungen der Geometrie des Implantatkerns vorgenommen werden. Insbesondere hat sich gezeigt, dass bei einem gezielt hinsichtlich der Geometrie abgestimmten Implantatkern gegenüber einem Implantatkern, dessen Artikulationsflächen bei zusammengesetztem Implantat vollflächig mit den Artikulationsflächen der Implantatplatten in Kontakt stehen, die Spitzenbelastungen um bis zu 30 % reduziert werden können. Abriebeffekte und Verschleißerscheinungen an den zusammenwirkenden Artikulationsflächen werden hierdurch spürbar reduziert.

Es hat sich insbesondere gezeigt, dass die gewünschten Belastungsreduzierungen durch eine verbesserte "Federwirkung" des über die Implantatplatten unter Druck gesetzten Implantatkerns erzielt werden können.

Ein Vorteil einer solchen Ausgestaltung besteht darin, dass mit Flüssigkeit gefüllte Hohlräume zwischen der Außenfläche des Implantatkerns und den Gegenflächen der Implantatplatten, die durch eine Berührung von Implantatkern und Implantatplatten abgedichtet sind, einen vorteilhaften hydrostatischen Stützeffekt bewirken bzw. unterstützen können, indem die wirksame Stützfläche auf den ganzen Innenbereich ausgedehnt wird.

In einem besonders bevorzugten praktischen Ausführungsbeispiel sind die Artikulationsflächen der Implantatplatten jeweils in Form einer Kugelteilfläche mit einem konstanten Krümmungsradius vorgesehen, wobei die Artikulationsflächen des Implantatkerns jeweils von mehreren unterschiedliche Krümmungsradien aufweisenden Kugelteilflächen gebildet sind. Bevorzugt werden die Artikulationsflächen der Implantatplatten jeweils von zwei Teilflächen gebildet, deren Krümmungsradien kleiner als der Krümmungsradius der Artikulationsflächen der Implantatplatten ist und die von einer Berührungslinie zwischen Implantatkern und Implantatplatten zum einen in Richtung des Kernpols und zum anderen in Richtung des Kernäquators ausgehen.

Alternativ oder zusätzlich kann vorgesehen sein, dass der Implantatkern insbesondere im Bereich seiner Äquatorialebene mit einer äußeren Ringnut und/oder mit einer inneren, bevorzugt eine radiale Erweiterung eines senkrecht zur Äquatorialebene verlaufenden Durchgangs bildenden Ringnut versehen ist.

Durch eine solche Materialwegnahme werden ebenfalls zu einer Reduzierung von Spitzenbelastungen führende Federbereiche geschaffen, aufgrund welcher sich der Implantatkern unter Druckeinwirkung in einer gezielt vorgebbaren Weise verformen kann.

Bevorzugt ist es, wenn der Implantatkern einen senkrecht zur Äquatorialebene verlaufenden Durchgang aufweist. Die vorstehend erwähnten Belastungsberechnungen haben gezeigt, dass sich durch die erläuterten Maßnahmen die Spitzenbelastungen unabhängig davon reduzieren lassen, ob ein solcher Durchgang vorhanden ist oder nicht. Dennoch wird durch einen solchen Durchgang eine weitere Möglichkeit zur Optimierung der Implantatgeometrie geschaffen.

Aufwändige Untersuchungen unter Zuhilfenahme von Modellrechnungen und Versuchen haben ferner ergeben, dass sich bestimmte räumliche Verteilungen der Nachgiebigkeit des Implantatkerns als besonders vorteilhaft erweisen. Durch eine geschickte Wahl der Abhängigkeit des elastischen Verhaltens bzw. der Federwirkung des Implantatkerns von dem radialen Abstand zu dessen Zentrum oder Mittelachse kann erreicht werden, dass an keiner Stelle der mit den Artikulationsflächen der Implantatplatten zusammenwirkenden Artikulationsflächen des Implantatkerns unakzeptierbar hohe spezifische Druckbelastungen auftreten. Insbesondere kann erreicht werden, dass Druckspitzen im radial äußeren Randbereich vermieden werden. Auf diese Weise kann erfolgreich einem Verschleiß der Artikulationsflächen entgegengewirkt werden, der die Gefahr eines in jedem Fall zu vermeidenden Materialabriebs mit sich bringt.

Gemäß einem bevorzugten Ausführungsbeispiel der Erfindung ist vorgesehen, dass der Implantatkern in einem radial äußeren Randbereich eine größere Nachgiebigkeit aufweist als in einem radial inneren Zentralbereich. Ferner kann vorgesehen sein, dass der Implantatkern in einem radial mittleren Bereich, der zwischen einem radial äußeren Bereich einerseits und einem zentralen, mit einem senkrecht zu einer Äquatorialebene verlaufenden Durchgang versehenen Bereich andererseits gelegen ist, die geringste Nachgiebigkeit und damit größte Steifigkeit aufweist.

Gemäßder Erfindung ist der Implantatkern mehrteilig ausgebildet. Es ist eine Anordnung aus wenigstens einem inneren Stützkissen und zumindest einer das Stützkissen umgebenden Schale vorgesehen. Das Stützkissen kann axiale Bewegungen der unmittelbar mit den Implantatplatten zusammenwirkenden Schale dämpfen. Beispielsweise durch die Art und Weise seiner inneren Abstützung oder durch seine Formgestaltung kann das Stützkissen insbesondere im radial äußeren Randbereich und - sofern vorhanden - im Bereich einer einen zentralen Durchgang begrenzenden Innenseite nachteilige Druckspitzen verhindern. Dieser mehrteilige Aufbau hat den Vorteil, dass auch bei für den Implantatkern verwendeten Materialien, die eine vergleichsweise geringe Verschleißfestigkeit aufweisen, das Entstehen von schädlichem Abrieb verhindert oder zumindest ausreichend stark reduziert werden.

Vorzugsweise weist das innere Stützkissen eine linsenförmige Grundform auf.

Ferner ist erfindungsgemäß vorgesehen, dass die Schale zwei Halbschalen umfasst, die bevorzugt in axialer Richtung mit Abstand voneinander angeordnet sind.

Des Weiteren ist bevorzugt vorgesehen, dass das Stützkissen und die Schale aus unterschiedlichen Materialien hergestellt sind. Dabei ist das Material der Schale vorzugsweise härter und/oder steifer als das Material des Stützkissens.

Ein besonders bevorzugtes Material für das Stützkissen ist Polycarbonaturethan (PCU). Dieses Material ist besonders gut dazu geeignet, einen gewünschten maximalen "Federweg" des Implantatkerns von etwa 1 mm zu erzielen. Alternativ kann als Material für das Stützkissen z.B. auch Silikon oder eine entsprechend den gewünschten elastischen Eigenschaften des Stützkissens eingestellte Mischung aus PCU und Silikon vorgesehen sein.

Obwohl es prinzipiell möglich ist, anstelle einer mehrteiligen Ausgestaltung des Implantatkerns diesen einstückig aus einem geeigneten Material wie insbesondere PCU herzustellen und übermäßige Druckbelastungen alleine durch eine geschickte Formgebung insbesondere in den axial äußeren Randbereichen zu verhindern, ist erfindungsgemäß vorgesehen, die Artikulationsflächen sozusagen zu "veredeln" und hierfür die erwähnte, das Stützkissen zumindest teilweise umgebende Schale bzw. die Halbschalen zu verwenden. Als Material für die Schale kommt vorzugsweise Polyethylen (PE), hochvernetztes Polyethylen, UHMWPE (UHMW = Ultra-High Molecular Weight) oder Metall, insbesondere eine CoCrMo- oder Titanlegierung, in Frage. Durch derartige Materialien kann insbesondere die Biokompatibilität gewährleistet werden.

Wenn das Stützkissen gemäß einer weiteren bevorzugten Ausgestaltung seine geringste Nachgiebigkeit bzw. größte Steifigkeit etwa in der Mitte zwischen dem radial äußeren Randbereich und einem zentralen Bereich aufweist, können nachteilige Stülpungen der Halbschalen, die bei Vorhandensein eines zentralen Durchgangs ringförmig ausgebildet sind, vermieden werden.

Ferner ist erfindungsgemäß vorgesehen, dass die Schale in radialer Richtung über das Stützkissen hinaus vorsteht. Durch diesen "Überhang" der Schale bzw. der beiden Halbschalen gegenüber dem inneren Stützkissen wird erreicht, dass die eigentliche Abstützung der Implantatplatten über die Schale bzw. Halbschalen in Richtung eines mittleren Bereiches zwischen axial äußerem Randbereich und zentralem Bereich verlagert wird und auf diese Weise Druckspitzen im Randbereich bzw. zentralen Bereich verhindert oder zumindest stark verringert werden.

Insbesondere im radial äußeren Randbereich des Implantatkerns kann zwischen der Schale bzw. den Halbschalen einerseits und dem Stützkissen andererseits jeweils ein Zwischenraum vorgesehen sein, so dass in diesem Bereich keine Abstützung der Schale am Stützkissen erfolgt.

Gemäß einem weiteren Ausführungsbeispiel der Erfindung ist zwischen dem Stützkissen und der Schale eine insbesondere aus Metall hergestellte Zwischenlage angeordnet. Der Verlauf dieser Zwischenlage kann grundsätzlich beliebig gewählt werden. So kann die Zwischenlage beispielsweise parallel zur Äquatorialebene verlaufen oder entsprechend der äußeren Schale gekrümmt sein.

Wenn eine derartige Zwischenlage vorhanden ist, die aus zwei separaten, jeweils einer Halbschale zugeordneten Einzellagen bestehen kann, dann kann weiter vorgesehen sein, dass sich die Schale bzw. die Halbschalen ausschließlich über diese Zwischenlage bzw. Einzellagen am inneren Stützkissen abstützen, d.h. eine Materialberührung findet ausschließlich zwischen der Schale und der Zwischenlage statt, nicht aber zwischen der Schale und dem Stützkissen.

Die Zwischenlage kann als Wegbegrenzung für in einen senkrecht zu einer Äquatorialebene verlaufenden Durchgang hineinragende Zapfen der Implantatplatten ausgebildet sein. Eine Beeinträchtigung der bevorzugt aus PE bestehenden äußeren Schale wird hierdurch in vorteilhafter Weise vermieden.

Des Weiteren ist vorzugsweise vorgesehen, dass ein senkrecht zu einer Äquatorialebene verlaufender Durchgang des Implantatkerns eine über seine Länge variierende Querschnittsfläche aufweist. Vorzugsweise nimmt die Querschnittsfläche jeweils von der Äquatorialebene nach außen insbesondere stetig zu. Durch die Formgestaltung des zentralen Durchgangs kann das Druckverhalten insbesondere des inneren Stützkissens gezielt eingestellt werden.

Eine weitere Möglichkeit, das Druckverhalten des Implantatkerns einzustellen, besteht gemäß einer weiteren bevorzugten Ausgestaltung der Erfindung darin, das Stützkissen in einem zentralen Bereich in axialer Richtung zu versteifen. Alternativ oder zusätzlich kann das Stützkissen im Fall des Vorsehens eines zentralen Durchgangs in radialer Richtung nach innen hin versteift werden.

Für die Versteifung des Stützkissens kann insbesondere ein separates, bevorzugt eine ringförmige oder zylindrische Grundform aufweisendes Versteifungselement vorgesehen sein. Dieses Versteifungselement kann im zentralen Durchgang angeordnet und beispielsweise als ein so genannter Metallfaltenbalg ausgebildet sein.

Ein derartiges Versteifungselement kann nicht nur im Zentralbereich bzw. am den zentralen Durchgang begrenzenden inneren Randbereich des Stützkissens dessen Steifigkeit erhöhen, sondern gleichzeitig auch das Stützkissen in radialer Richtung abstützen, wodurch die Steifigkeit des Stützkissens im zentralen Bereich ebenfalls vergrößert wird.

Ein beispielsweise als Metallfaltenbalg ausgebildetes Versteifungselement bietet außerdem die vorteilhafte Möglichkeit, die das Stützkissen zumindest teilweise umgebenden, im Fall eines zentralen Durchgangs ringförmig ausgebildeten Halbschalen besser zu führen, wodurch ein "Schwimmen" der Halbschalen auf dem Stützkissen vermieden wird.

In einer bevorzugten Ausgestaltung der Erfindung ist das Stützkissen an die Schale bzw. die Halbschalen angespritzt, wobei vorzugsweise zur Bildung eines durch das Anspritzen herstellbaren Materialverbundes zwischen Stützkissen und Schale das Material des Stützkissens einen höheren Schmelzpunkt aufweist als das Material der Schale. Auf die Herstellung des erfindungsgemäßen Bandscheibenimplantats wird an anderer Stelle näher eingegangen.

Auch bei Verwendung einer Zwischenlage, wie sie vorstehend erläutert wurde, kann vorgesehen sein, dass das Stützkissen an die Zwischenlage angespritzt ist.

Des Weiteren wird erfindungsgemäß vorgeschlagen, das Stützkissen oder eine mit dem Stützkissen verbundene, insbesondere aus Metall hergestellte Zwischenlage mit der Schale bzw. den Halbschalen durch eine Clips-, Schnapp- oder Rastverbindung zu verbinden.

Was die Implantatplatten des erfindungsgemäßen Implantats anbetrifft, so ist erfindungsgemäß bevorzugt vorgesehen, dass die Implantatplatten jeweils auf ihrer Außenseite eine domförmige Erweiterung insbesondere in Form eines Kugelsegments oder eine tonnenförmige Erweiterung aufweisen. Diese Dome bzw. Tonnen sorgen für eine primäre Positionsstabilität des Implantats nach dem Einsetzen, wobei eine tonnenförmige Erweiterung außerdem während des Einsetzens eine Führungsfunktion erfüllen kann.

Des Weiteren wird erfindungsgemäß vorgeschlagen, dass die Außenseiten der Implantatplatten jeweils nach außen gewölbt sind. Diese Wölbungen sind bevorzugt zusätzlich zu den vorstehend erwähnten dom- oder tonnenförmigen Erweiterungen vorgesehen, und zwar derart, dass jeweils die Wölbung flacher ist, dafür jedoch in der Plattenebene eine größere Ausdehnung aufweist als der Dom bzw. die Tonne.

Des Weiteren kann erfindungsgemäß vorgesehen sein, dass die Außenseiten der Implantatplatten jeweils einen sich zumindest über einen Teil des Umfangs der Implantatplatten erstreckenden ebenen Randbereich aufweisen.

Insgesamt lässt sich durch eine Ausgestaltung der Außenseiten der Implantatplatten jeweils mit vergleichsweise stark gekrümmter dom- bzw. tonnenförmiger Erweiterung, relativ flacher Wölbung und ebenem Randbereich ein konturoptimiertes Interface zum knöchernen Aufbau des Wirbelkörpers erzielen.

Ferner können die Implantatplatten jeweils auf ihrer Außenseite wenigstens einen insbesondere als Finne ausgebildeten Führungsvorsprung und/oder einen insbesondere pyramidenförmigen Haltevorsprung aufweisen. Hierdurch wird dem Implantat im eingesetzten Zustand Rotationsstabilität verliehen, wobei die Haltevorsprünge dem eingesetzten Implantat zusätzlich eine Herausrutschsicherheit verleihen können.

In einer besonders bevorzugten Ausführungsform ist vorgesehen, dass die Implantatplatten jeweils auf ihrer Innenseite eine Vertiefung zur Aufnahme des Implantatkerns aufweisen, wobei die zusammenwirkenden Artikulationsflächen der Vertiefung und des Implantatkerns jeweils Kugelteilflächen sind. Die Vertiefungen ermöglichen eine versenkte und damit herausrutschsichere Anordnung des Implantatkerns zwischen den Implantatplatten. Durch die Ausbildung der Artikulationsflächen als Teilflächen einer Kugel ist das erfindungsgemäße Bandscheibenimplantat hinsichtlich seiner Bewegungsmöglichkeiten rotationssymmetrisch.

Um ein Herausrutschen des Implantatkerns aus dem durch die Vertiefungen oder Konkavitäten der Implantatplatten gebildeten Aufnahmeraum bei extremen Körperhaltungen sicher zu verhindern, kann vorgesehen sein, dass zumindest eine Implantatplatte einen von ihrer Innenseite abstehenden Zapfen aufweist, der bei zusammengesetztem Implantat in eine auf der Außenseite des Implantatkerns ausgebildete Ausnehmung hineinragt, wobei die Ausnehmung größer als der Zapfen bemessen ist, um eine Relativbewegung zwischen Implantatplatte und Implantatkern zu ermöglichen.

Der Zapfen und/oder das Zentrum des Implantatkerns können sowohl mittig als auch exzentrisch bezüglich der Abmessung der Implantatplatte in sagittaler Richtung angeordnet sein.

Um die benötigte Distraktionshöhe zum Einbringen des Implantatkerns zwischen die Implantatplatten möglichst gering zu halten, kann gemäß einem weiteren Ausführungsbeispiel vorgesehen sein, dass der Implantatkem auf zumindest einer Außenseite mit einem vom Rand zur Ausnehmung verlaufenden Einführkanal für den Zapfen der Implantatplatte versehen ist.

Eine alternative oder zusätzliche Möglichkeit, die Distraktionshöhe gering zu halten, besteht gemäß einer weiteren Ausführungsform darin, zumindest eine Implantatplatte auf ihrer Innenseite mit einem vom Rand zur Vertiefung verlaufenden Einführkanal für den Implantatkern zu versehen.

Die Erfindung betrifft außerdem ein Verfahren zur Herstellung eines Bandscheibenimplantats nach Anspruch 1, wobei das Stützkissen in einem Kunststoffspritzverfahren an die Schale, insbesondere an die Halbschalen, oder an eine im fertigen Zustand zwischen dem Stützkissen und der Schale angeordnete Zwischenlage angespritzt wird.

Vorzugsweise wird zur Herstellung eines Materialverbundes zwischen Stützkissen und Schale ein Material für das anzuspritzende Stützkissen gewählt, das einen höheren Schmelzpunkt aufweist als das Material der Schale. Wie vorstehend bereits erwähnt, ist ein bevorzugtes Material für das Stützkissen Polycarbonaturethan (PCU), Silikon oder eine Mischung aus PCU und Silikon, während für die Schale vorzugsweise Polyethylen (PE), hochvernetztes PE, UHMWPE oder Metall verwendet wird. Während der Schmelzpunkt von PCU oberhalb von 200°C gelegen ist, liegt der Schmelzpunkt von PE im Bereich von 120°C. Es wurde gefunden, dass sich dennoch aus PE hergestellte Halbschalen unter Verwendung einer gekühlten Spritzform derart mit PCU ausspritzen lassen, dass ein geeigneter Materialverbund entsteht.

Dieser Materialverbund kann dadurch verbessert werden, dass an der Innenseite der Halbschalen ausgebildete Rücksprünge oder Hinterschneidungen beim Anspritzen des Stützkissen-Materials ausgefüllt werden.

Die Erfindung wird im Folgenden beispielhaft unter Bezugnahme auf die Zeichnung beschrieben. Die Fig. 1 bis 6, Fig. 11c und Fig. 12 zeigen Bandscheibenimplantate, die nicht zu der Erfindung gehören, deren Beschreibung jedoch für das Verständnis der Erfindung hilfreich ist. Es zeigen:
- Fig. 1: verschiedene Ansichten eines Bandscheibenimplantats,
- Fig. 2a+2b: verschiedene perspektivische Ansichten des Bandscheibenimplantats von Fig. 1,
- Fig. 2c: eine hinsichtlich der Implantatplatten alternative Ausführung des Bandscheibenimplantats von Fig. 1,
- Fig. 3a-3c: jeweils eine Draufsicht auf eine gegenüber Fig. 1 modifizierte Ausführungsform eines Implantatkerns,
- Fig. 4: eine perspektivische Ansicht eines gegenüber Fig. 1 modifizierten Bandscheibenimplantats,
- Fig. 5: ein weiteres Ausführungsbeispiel eines Bandscheibenimplantats,
- Fig. 6: ein weiteres Ausführungsbeispiel eines Bandscheibenimplantats,
- Fig. 7 - 11: Ausführungsbeispiele eines erfindungsgemäßen Bandscheibenimplantats, und

- Fig. 12: eine weitere Ausführungsform eines Bandscheibenimplantats.

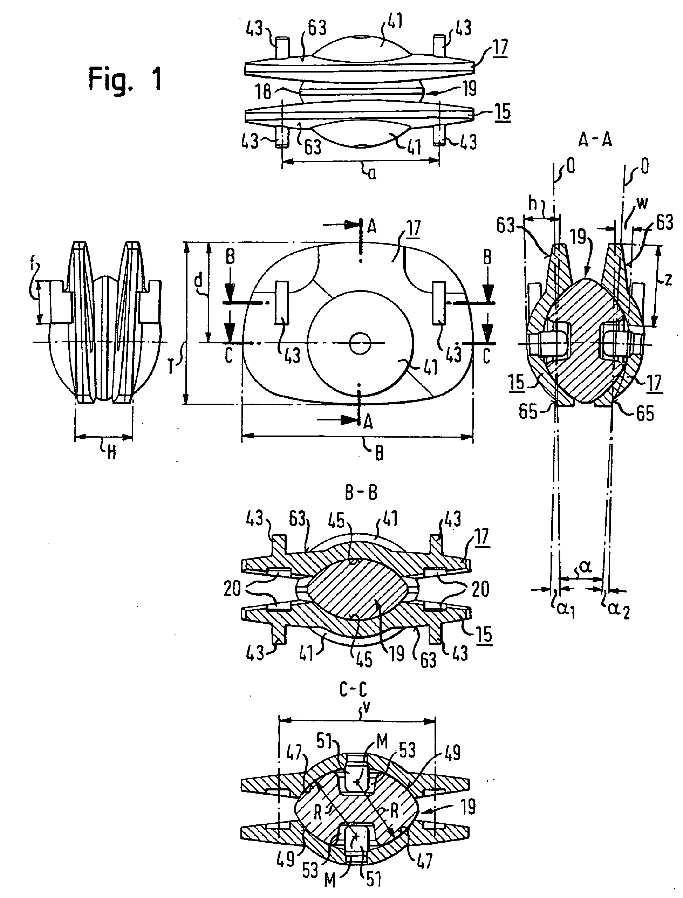

Fig. 1 zeigt verschiedene Ansichten eines Bandscheibenimplantats, das zwei auch als Deckplatten oder Endplatten bezeichnete Implantatplatten 15, 17 sowie einen auch als Inlay bezeichneten Implantatkern 19 umfasst. Wie im Einleitungsteil bereits erwähnt, wird in dieser Anmeldung auf das Einsetzen des Bandscheibenimplantats nicht näher eingegangen. Die ebenfalls bereits erwähnte europäische Patentanmeldung EP 1 532 948 beschreibt eine insbesondere für die Bandscheibenimplantate gemäß den Fig. 1 bis 4 geeignete Aufspreizeinrichtung, von der nachstehend einige Bestandteile erwähnt werden, soweit es für das Verständnis der in den Fig. 1 bis 4 beschriebenen Implantate erforderlich ist.

Der Implantatkern 19 besitzt eine linsenartige Grundform, die zwei mit ihren ebenen Seiten aneinander liegenden Kugelsegmenten entspricht. Die äußeren Artikulationsflächen 49 des Implantatkerns 19 sind somit Kugelteilflächen. Wie insbesondere der oberen Seitenansicht in Fig. 1 zu entnehmen ist, entspricht die Form des Implantatkerns 19 nicht exakt zwei aufeinander gesetzten Kugelsegmenten, sondern es befindet sich zwischen den ebenen Seiten der Kugelsegmente eine Zwischenscheibe 18 von relativ geringer Höhe und mit geradem Rand.

An seinen Polen ist der Implantatkern 19 mit Ausnehmungen 53 versehen, in die bei zusammengesetztem Implantat Zapfen 51 der Implantatplatten 15, 17 hineinragen, worauf nachstehend näher eingegangen wird.

Wie insbesondere den Schnitten B-B und C-C zu entnehmen ist, sind die Implantatplatten 15, 17 jeweils auf ihrer Außenseite mit einer relativ flachen Wölbung 63 versehen, auf der sich wiederum eine stärker gekrümmte domförmige Erweiterung 41 erhebt, die einer Vertiefung 45 auf der Innenseite der Implantatplatte 15, 17 entspricht, deren Artikulationsfläche 47 ebenfalls eine Teilfläche einer Kugel ist, deren Radius demjenigen der Artikulationsflächen 49 des Implantatkerns 19 entspricht. Wie insbesondere der Schnitt C-C zeigt, besteht im zusammengesetzten Zustand des Implantats vollflächiger Kontakt zwischen den beiden Artikulationsflächen 47, 49. Für jedes Kugelsegment des Implantatkerns 19 liegt der Mittelpunkt M der Kugel, auf deren Oberfläche die Artikulationsflächen 47, 49 liegen, innerhalb des jeweils anderen Kugelsegmentes, und zwar im Bereich der Ausnehmung 53.

Auf ihren Außenseiten sind die Implantatplatten 15, 17 des Weiteren mit Finnen 43 versehen. Mit diesen Führungsvorsprüngen 43 werden die Implantatplatten 15, 17 beim Einsetzen in ein Bandscheibenfach in zuvor mittels eines Finnenschlägers vorbereiteten nutförmigen Ausnehmungen auf den Oberflächen der Wirbelkörper geführt.

Den Finnen 43 gegenüberliegend sind auf den Innenseiten der Implantatplatten 15, 17 Aussparungen 20 zur Aufnahme eines Adapterelementes eines Distraktionsschuhs ausgebildet.

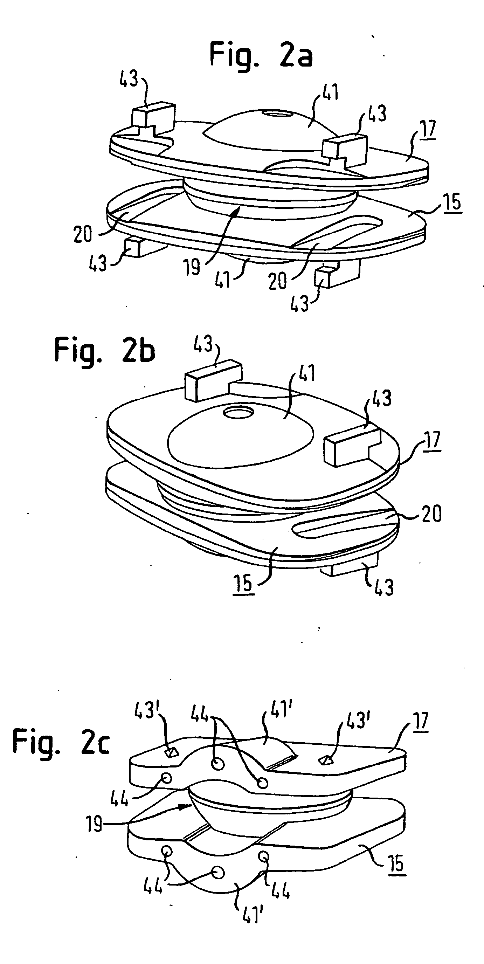

Die Variante gemäß Fig. 2c unterscheidet sich von dem in Fig. 1 und Fig. 2a und 2b dargestellten Implantat durch die Ausgestaltung der Außenseiten der Implantatplatten 15, 17, die hier jeweils mit einer tonnenförmigen Erweiterung 41' versehen sind, wodurch im eingesetzten Zustand wiederum eine Positionsstabilität der Implantatplatten 15, 17 und zusätzlich beim Einsetzen der Implantatplatten 15, 17 eine Längsführung gegeben ist.

Anstelle von z.B. finnenartigen Führungselementen sind außerdem zackenartige, eine Pyramidenform aufweisende Haltevorsprünge 43' vorgesehen. Die Höhe dieser auch als Stifte oder Pins bezeichneten, spitz zulaufenden Vorsprünge 43' ist derart gewählt, dass sie das Einsetzen der Implantatplatten 15, 17 nicht nachteilig beeinflussen, bei eingesetztem Implantat jedoch für eine Positionsfixierung sorgen, indem sie in die einander zugewandten Wirbelkörperflächen eingreifen. Ein optimales Einsetzverhalten wird dadurch erzielt, dass jeweils eine Kante der pyramidenförmigen Pins 43' in Einsetzrichtung weist.

Das Implantat gemäß Fig. 2c ist für ein anderes Operationsverfahren und insbesondere für eine andere Art und Weise des Einsetzens der Implantatplatten 15, 17 sowie deren Aufspreizen konzipiert als das Implantat gemäß Fig. 1 und Fig. 2a und 2b. Insbesondere kommen andere Instrumente zum Einsatz, auf die in der vorliegenden Anmeldung nicht näher eingegangen wird. Diesbezüglich wird auf die europäische Patentanmeldung EP 1 647 244 Bezug genommen. Zur Verwendung mit den in der genannten Anmeldung beschriebenen Instrumenten, insbesondere Setzgeräten, sind die Implantatplatten 15, 17 jeweils an ihrer ventralen Seite mit Bohrungen 44 zur Aufnahme von entsprechenden Vorsprüngen der Setzgeräte versehen.

Der Durchmesser der in Form von separaten Elementen vorgesehenen Zapfen 51 der Implantatplatten 15, 17 (Schnitt C-C in Fig. 1) ist kleiner als derjenige der im Implantatkern 19 ausgebildeten Ausnehmungen 53. Die auf diese Weise mit Spiel in die Ausnehmungen 53 hineinragenden Zapfen 51 verhindern ein Herausrutschen des Implantatkerns 19 aus dem von den beiden Vertiefungen 45 gebildeten Aufnahmeraum bei extremen Körperhaltungen.

Wie insbesondere der Schnitt A-A in Fig. 1 zeigt, verlaufen die flachen Wölbungen 63 auf den Außenseiten der Implantatplatten 15, 17 jeweils nicht über den gesamten Umfang bis zum Plattenrand. Über einen Teilumfang der Implantatplatten 15, 17 erstreckt sich ein ebener Randbereich 65.

Der Schnitt A-A zeigt außerdem, dass die so genannte Angulation der Implantatplatten 15, 17 jeweils bezüglich einer Nullreferenz 0 gemessen wird, bei der es sich um eine Ebene handelt, die senkrecht zu den gestrichelt eingezeichneten Mittelachsen der Zapfen 51 verläuft. Der resultierende Angulationswinkel α des zusammengesetzten Implantats bei einer bestimmten Relativstellung zwischen dem Implantatkern 19 und den beiden Implantatplatten 15, 17 wird bestimmt durch die Summe aus der kaudalen Angulation α1 und der kranialen Angulation α2.

Aus der Draufsicht und aus Schnitt A-A ist ersichtlich, dass das Zentrum von Dom 41 und Zapfen 51 exzentrisch längs der Mittellinie nach posterior verschoben ist.

Das Bandscheibenimplantat weist bestimmte charakteristische Größen auf, die bei der Implantatherstellung zur Optimierung des Implantats und zur Anpassung an die jeweilige Patientenanatomie variiert werden können. Hierbei handelt es sich insbesondere um die folgenden Parameter, deren Definition jeweils den verschiedenen Ansichten der Fig. 1 entnommen werden kann:
- H: Höhe des Implantats
- B: Breite des Implantats
- T: Tiefe des Implantats
- R: Radius der Artikulationsflächen
- d: Domposition
- h: Domhöhe
- z: Wölbungszentrum
- w: Wölbungshöhe
- a: Finnenabstand
- f: Finnenhöhe
- v: Abstand der Aussparungen

Entsprechende Parameter existieren analog auch für die Variante der Fig. 2c, bei der sich folglich jeweils die Parameter d und h auf die Tonne 41' und die Parameter a und f auf die Position bzw. den Abstand und auf die Höhe der Pins 43' beziehen und der Parameter v den Abstand zwischen den beiden äußeren Bohrungen 44 für das Einsetzinstrument angibt.

Abweichend von dem in Fig. 1 dargestellten Beispiel können die Zapfen 51 auch weggelassen werden. Eine derartige alternative Ausgestaltung kommt insbesondere dann in Frage, wenn die Vertiefungen 45 in den Implantatplatten 15, 17 derart ausgebildet sind oder ausgebildet werden können, dass sie alleine bereits eine ausreichende Extrusionssicherheit bietet, d.h. mit ausreichender Sicherheit ein Herausrutschen des Implantatkerns 19 verhindern.

Die Implantatplatten 15, 17 können aus einer CoCr-Legierung oder einer Titan-Legierung hergestellt und auf der äußeren Knochenseite mit porösem Titan und gegebenenfalls zusätzlich mit Hydroxy-Apatit (HAC) beschichtet sein, um auf diese Weise ein besonders schnelles Anwachsen des Knochens zu ermöglichen. In der Praxis steht bevorzugt ein Satz von unterschiedlich großen Implantatplatten 15, 17 zur Verfügung, um eine optimale Anpassung an verschiedene Patientenanatomien zu erreichen. Die Implantatplatten 15, 17 können sich insbesondere hinsichtlich ihrer Breite, Tiefe und Winkelung (Angulation) voneinander unterscheiden.

Der Implantatkern 19 kann beispielsweise aus Polyethylen, hochvernetztem PE, UHMWPE oder Metall, insbesondere einer CoCrMo-Legierung, bestehen. Dabei ist Polyethylen das bevorzugte Material, da hierdurch axial einwirkende Kräfte besser elastisch aufgenommen werden können, d.h. ein besseres axiales Dämpfungsvermögen vorhanden ist. Um einen eventuell möglichen Abrieb zu vermeiden, kann eine dünne Metallschale über das Kunststoffmaterial gelegt werden. Es entsteht dann eine Kombination von metallischen Kugelteilflächen, die sich aufgrund ihrer Kugelform in enorm hoher Präzision zueinander herstellen lassen. Ein derartiges Metall-Metall-Zusammenspiel ist allgemein in der europäischen Patentanmeldung 97903208.3 (Veröffentlichungsnummer EP 0 892 627) beschrieben, auf deren Inhalt hiermit zur Ergänzung der Offenbarung der vorliegenden Anmeldung ausdrücklich Bezug genommen wird.

Durch die versenkte Anordnung des Implantatkerns 19 in den Konkavitäten 45 der Implantatplatten 15, 17 wird eine relativ große Kraftübertragungsfläche bereitgestellt und damit eine vergleichsweise kleine Flächenlast erreicht, wobei gleichzeitig die Extrusionsgefahr gering gehalten wird.

Die perspektivischen Darstellungen des Implantats in den Fig. 2a und 2b zeigen insbesondere die auf den Innenseiten der Implantatplatten 15, 17 ausgebildeten Aussparungen 20 für die Distraktionsschuhe und die Ausgestaltung der Außenseiten der Implantatplatten 15, 17 mit dem Dom 41 und den Finnen 43.

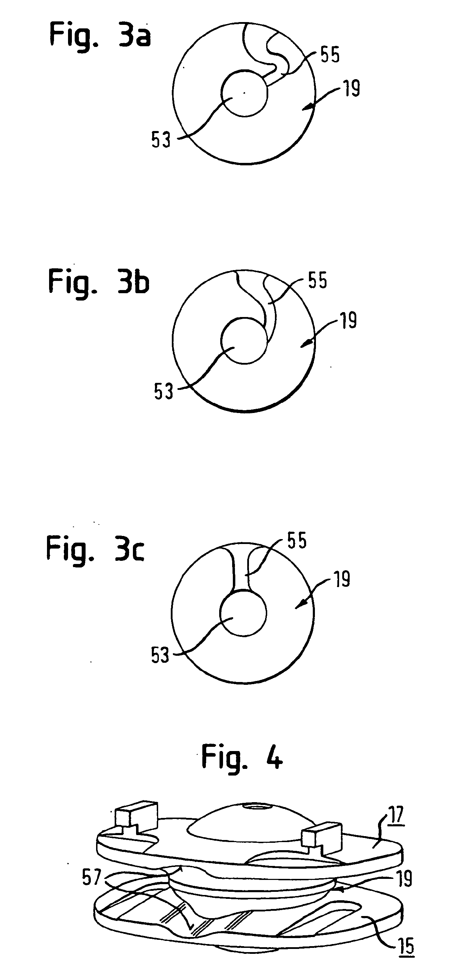

Die Fig. 3a-3c sowie Fig. 4 zeigen mögliche Maßnahmen, die am Implantatkem 19 (Fig. 3a-3c) und an den Innenseiten der Implantatplatten 15, 17 (Fig. 4) getroffen werden können, um das Maß, um welches die Implantatplatten 15, 17 zum Einführen des Implantatkerns 19 auseinander gedrückt werden müssen, möglichst gering zu halten.

Gemäß Fig. 3a-3c ist auf der Außenseite des Implantatkerns 19 jeweils ein sich vom Rand des Implantatkerns 19 bis zu der zentralen Ausnehmung 53 erstreckender Einführkanal 55 ausgebildet. Der Einführkanal 55 kann grundsätzlich einen beliebig gekrümmten Verlauf aufweisen und dabei entweder im Wesentlichen radial (Fig. 3a) oder tangential (Fig. 3b) in die Ausnehmung 53 münden. Alternativ kann der Einführkanal 55 einen geradlinigen radialen Verlauf aufweisen (Fig. 3c).

Beim Einführen des Implantatkerns 19 zwischen die Implantatplatten 15, 17 ragen die Zapfen 51 in die Einführkanäle 55 des Implantatkerns 19 hinein, so dass die Zapfen 51 auch bei geringerem Plattenabstand dem einzuführenden Implantatkern 19 nicht im Wege sind.

Alternativ oder zusätzlich zu den Einführkanälen 55 des Implantatkerns 19 können die Implantatplatten 15, 17 jeweils auf ihrer Innenseite mit einem Einführkanal 57 in Form einer rinnenartigen Ausnehmung versehen sein, die von dem anterioren Plattenrand bis zur Vertiefung 45 verläuft, wodurch insgesamt ein sich von der anterioren Seite bis zum Aufnahmeraum für den Implantatkern 19 erstreckender "Einführtunnel" für den Implantatkern 19 vorhanden ist. Der Implantatkern 19 ist bereits zu Beginn des Einführvorgangs teilweise in die Einführkanäle 57 aufgenommen, so dass die Implantatplatten 15, 17 weniger weit auseinander gedrückt zu werden brauchen.

Bei einer Operation zum Einsetzen des Bandscheibenimplantats erfolgt die Vorbereitung des Bandscheibenfaches bis zu dem Zeitpunkt, in dem ein bestimmtes Operationssystems zum Einsatz kommt, wie bisher, d.h. das Räumen der natürlichen Zwischenwirbelscheibe erfolgt ohne das bestimmte Operationssystem. Auch eine erste Vorbereitung der Endplatten der Wirbelkörper, insbesondere mit einem so genannten "scharfen Löffel" (z.B. Cobb), erfolgt ohne Verwendung der Arbeitsplatten 11, 13.

Im Anschluss an diese erste Vorbereitung des Bandscheibenfaches kann ein Operationssystem beispielsweise derart eingesetzt werden, wie es in der vorstehend bereits erwähnten europäischen Patentanmeldung EP 03 026 582 beschrieben ist.

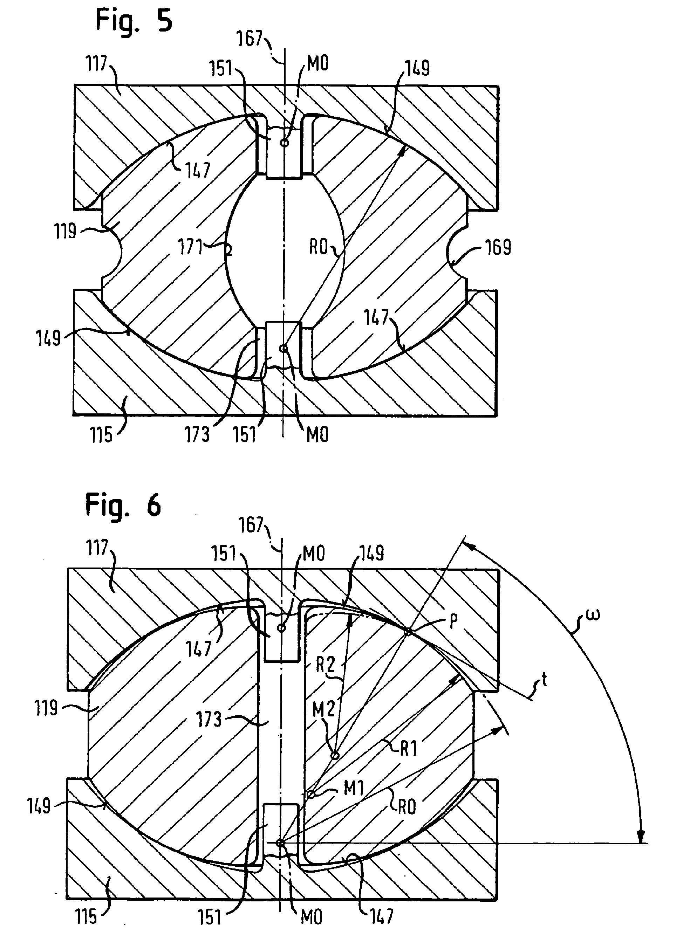

Die Fig. 5 und 6 zeigen bevorzugte Ausführungsbeispiele für ein Bandscheibenimplantat. Gemeinsam ist beiden Ausführungsformen, dass die Artikulationsflächen 147 der Implantatplatten 115, 117 jeweils Kugelteilflächen mit einem Radius R0 und einem auf der Mittelachse 167 des Implantats sowie auf einem Zapfen 151 der jeweils anderen Platte liegenden Mittelpunkt M0 sind. Beide Implantatkerne 119 sind zudem jeweils rotationssymmetrisch ausgebildet und mit einem zentralen Durchgang 173 versehen, dessen Längsachse mit der Mittelachse 167 zusammenfällt.

Bei dem Implantatkern 119 gemäß Fig. 5 sind die Artikulationsflächen 149 ebenfalls Kugelteilflächen mit einem Radius R0 und einem Mittelpunkt M0 entsprechend den Artikulationsflächen 147 der Implantatplatten 115, 117, so dass analog zu dem Implantat gemäß Fig. 1 der Implantatkern 119 und die Implantatplatten 115, 117 mit ihren Artikulationsflächen 147, 149 vollflächig aneinander liegen.

Um zur Minimierung von Spitzenbelastungen unter Druckeinwirkung einen verbesserten "Federeffekt" zu erzielen, wie er im Einleitungsteil erläutert ist, ist der Implantatkern 119 in Höhe der Äquatorialebene mit einer äußeren Ringnut 169 und einer im Vergleich zur äußeren Ringnut 169 wesentlich breiteren inneren Ringnut 171 versehen, die insofern eine radiale Erweiterung des zentralen Durchgangs 173 darstellt.

Bei dem Implantatkern 119 gemäß Fig. 6 wurde ein anderer Ansatz zur Erzielung einer verbesserten Stützwirkung gewählt. Hier sind die Artikulationsflächen 149 des Implantatkerns 119 keine entsprechend den Artikulationsflächen 147 der Implantatplatten 115, 117 geformten Kugelteilflächen. Vielmehr ist in jedem Quadranten die Artikulationsfläche 149 des Implantatkerns 119 derart geformt, dass sich der Implantatkern 119 und die Implantatplatten 115, 117 lediglich an einer Linie P berühren. In dem hier dargestellten Querschnitt längs der Mittelachse 167 ist die Lage der Berührungslinie P derart gewählt, dass eine durch den Mittelpunkt M0 und den Punkt P verlaufende, also die Tangente t durch den Punkt P rechtwinklig schneidende Gerade mit der Äquatorialebene des Implantatkerns 119 einen Winkel ω einschließt, der etwa 60° beträgt. Bevorzugt liegt der Winkel ω in einem Winkelbereich von etwa 45° bis 75°.

Basierend auf diesem Grundprinzip einer Linienberührung zwischen dem Implantatkern 119 und den Implantatplatten 115, 117 zeigt Fig. 6 zwei bevorzugte Varianten. In der mit durchgezogenen Linien dargestellten Variante besitzt die Artikulationsfläche 149 des Implantatkerns 119 einen konstanten Krümmungsradius R 1 < R0 mit einem Mittelpunkt M1. Mit der doppelt strichpunktierten Linie ist in Fig. 6 eine Variante gezeigt, bei der ausgehend von der Berührungslinie P die Krümmung der Artikulationsfläche 149 des Implantatkerns 119 in Richtung des Kernpols größer ist als in Richtung des Kernäquators, d.h. der Krümmungsradius R2 mit Mittelpunkt M2 ist kleiner als der Krümmungsradius R1 mit Mittelpunkt M1.

Eine bevorzugte Bedingung für diese Parameter lautet R0 - 6 mm < R1 < R0 - 1 mm, wobei R2 < R1 und 8 mm < R0 < 18 mm.

Gemäß Fig. 6 ist in diesem Ausführungsbeispiel ferner vorgesehen, dass die Mittelpunkte M0, M1 und M2 auf einer gemeinsamen Geraden liegen, welche die den Übergang zwischen den beiden Artikulationsflächenregionen des Implantatkerns 119 markierende Berührungslinie P schneidet.

Erfindungsgemäß ist auch eine Kombination der speziellen Artikulationsflächen-Geometrie gemäß Fig. 6 mit dem Ringnuten-Ansatz gemäß Fig. 5 prinzipiell möglich, d.h. verschiedene Maßnahmen, die jeweils zu einer von einer einfachen Grundform abweichenden Geometrie des Implantatkerns führen, können zur Erzielung eines verbesserten "Federeffekts" grundsätzlich miteinander kombiniert werden.

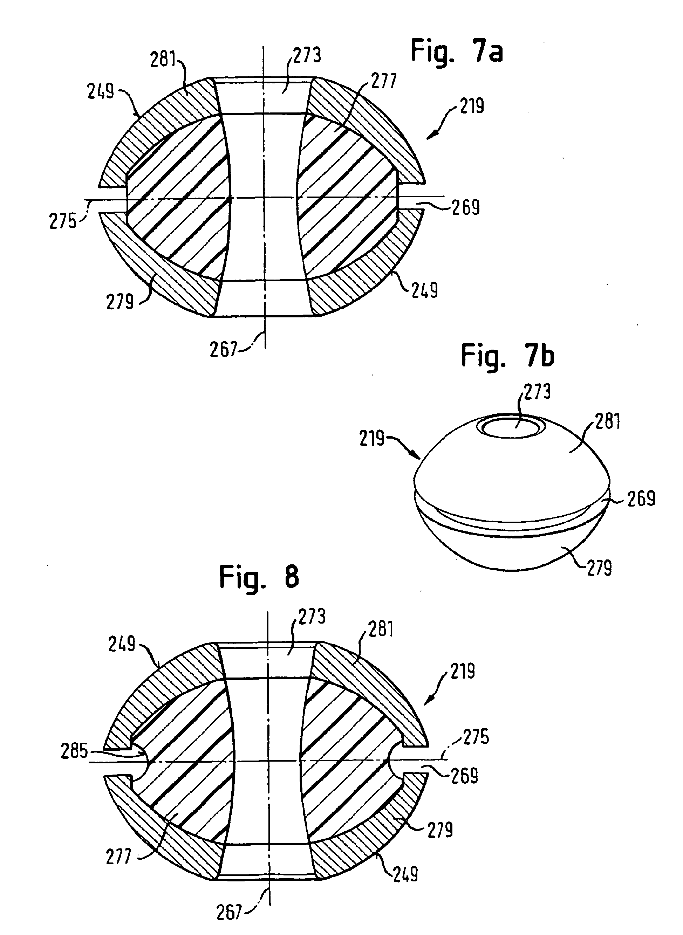

Die in dieser Anmeldung und insbesondere nachstehend in Verbindung mit den Fig. 7 bis 12 beschriebenen Implantatkerne - und damit auch die anhand von Fig. 7 bis 11 beschriebenen erfindungsgemäßen Implantatkerne - sind insbesondere hinsichtlich ihrer Abmessungen auf einen durchschnittlichen Mitteleuropäer abgestimmt. Die Implantatkerne besitzen eine Linsenform mit einem Außendurchmesser von etwa 25 mm und einer Höhe von etwa 19 mm, die durch die Abflachung eines zentralen, in axialer Richtung verlaufenden Durchgangs gegeben ist. Ferner sind die Implantatkerne für einen Krümmungsradius R0 von etwa 14 mm der in den Fig. 7 bis 12 nicht dargestellten Implantatplatten konzipiert.

Allen Implantatkernen ist ferner gemeinsam, dass die mit Artikulationsflächen der Implantatplatten zusammenwirkenden Artikulationsflächen des Implantatkerns Kugelteilflächen mit dem erwähnten Krümmungsradius R0 von etwa 14 mm sind. Die Linsenform der Implantatkerne ist dadurch selbst ausrichtend, dass die Implantatkerne zumindest näherungsweise die Form zweier mit ihren ebenen Seiten einander zugewandter Kugelsegmenten aufweisen, wobei jeweils der Kugelmittelpunkt des einen Kugelsegments innerhalb des anderen Kugelsegments liegt.

Der erfindungsgemäße Implantatkern gemäß Fig. 7a und 7b umfasst ein etwa linsenförmiges Stützkissen 277, auf dem zwei mit Abstand einander gegenüberliegende Halbschalen 279, 281 angeordnet sind. Das Stützkissen 277 besteht aus Polycarbonaturethan (PCU), Silikon oder einer PCU/Silikon-Mischung, während die beiden Halbschalen 279, 281 aus Polyethylen (PE), hochvernetztem PE, UHMWPE oder Metall, insbesondere einer CoCrMo-Legierung, hergestellt sind. Aufgrund eines senkrecht zur Äquatorialebene 275 verlaufenden zentralen Durchgangs 273, dessen Mittelachse mit der Mittelachse 267 des Implantatkerns 219 zusammenfällt, wiesen sowohl das Stützkissen 277 als auch die beiden Halbschalen 279, 281 jeweils eine Ringform auf.

In radialer Richtung stehen die Halbschalen 279, 281 über das Stützkissen 277 hinaus vor. Im Bereich dieses Überhangs bzw. dieser Überdeckung ist zwischen den insofern axial beabstandeten Halbschalen 279, 281 ein Ringspalt vorhanden, der eine radial äußere Ringnut 269 des Implantatkerns 219 bildet. In der perspektivischen Darstellung der Fig. 7b ist insbesondere diese Ringnut 269 zu erkennen.

Die von den Außenseiten der Halbschalen 279, 281 gebildeten Artikulationsflächen 249 des Implantatkerns 219 sind Kugelteilflächen und besitzen den gleichen Krümmungsradius wie die Artikulationsflächen der nicht dargestellten Implantatplatten des Bandscheibenimplantats.

Der radial äußere Seitenrand des Stützkissens 277 verläuft parallel zur Mittelachse 267, wohingegen der den zentralen Durchgang 273 begrenzende innere Rand oder die Innenseite des Stützkissens 277 konvex geformt ist. Dieser Verlauf der Innenseite des Stützkissens 277 wird durch den inneren Randbereich der Halbschalen 279, 281 fortgesetzt. Der zentrale Durchgang 273 besitzt folglich in dem hier dargestellten Axialschnitt eine doppelkonus-, doppeltrichter- oder sanduhrartige Form mit einer in der Äquatorialebene 275 minimalen freien inneren Querschnittsfläche.

In den zentralen Durchgang 273 ragen im zusammengesetzten Zustand des Bandscheibenimplantats z.B. entsprechend den Ausführungsformen der Fig. 5 und 6 Zapfen der Implantatplatten hinein.

Das Stützkissen 277 und die beiden Halbschalen 279, 281 bilden einen festen Materialverbund, der durch Anspritzen des für das Stützkissen 277 verwendeten Materials (insbesondere PCU, Silikon oder eine PCU/Silikon-Mischung) an die insbesondere aus PE, hochvernetztem PE, UHMWPE oder Metall, insbesondere einer CoCrMo-Legierung, bestehenden Halbschalen 279, 281 hergestellt wird, wie es im Einleitungsteil beschrieben ist.

Aufgrund seiner Linsenform bietet das Stützkissen 277 für die Halbschalen 279, 281 in seinem zentralen Bereich eine weichere Abstützung in axialer Richtung als im radial äußeren Randbereich. Dieses Verhalten kann durch die Formgebung des zentralen Durchgangs 273 beeinflusst werden. Durch den erwähnten radialen Überstand der Halbschalen 279, 281 wird die eigentliche Abstützung nach radial innen verlagert, wodurch das Auftreten von Druckspitzen im radial äußeren Randbereich vermieden wird.

Der Implantatkern 219 gemäß Fig. 8 unterscheidet sich von demjenigen der Fig. 7a und 7b durch das Vorsehen einer im Stützkissen 277 ausgebildeten äußeren Ringnut 285 in Höhe der Äquatorialebene 275. Durch eine derartige Einschnürung lässt sich der Verlauf der Abnahme der axialen Höhe des Stützkissens 277 im radial äußeren Randbereich einstellen.

Zusammen mit dem äquatorialen Ringspalt zwischen den beiden Halbschalen 279, 281 bildet die Ringnut 285 des Stützkissens 277 die äußere Ringnut 269 des gesamten Implantatkerns 219.

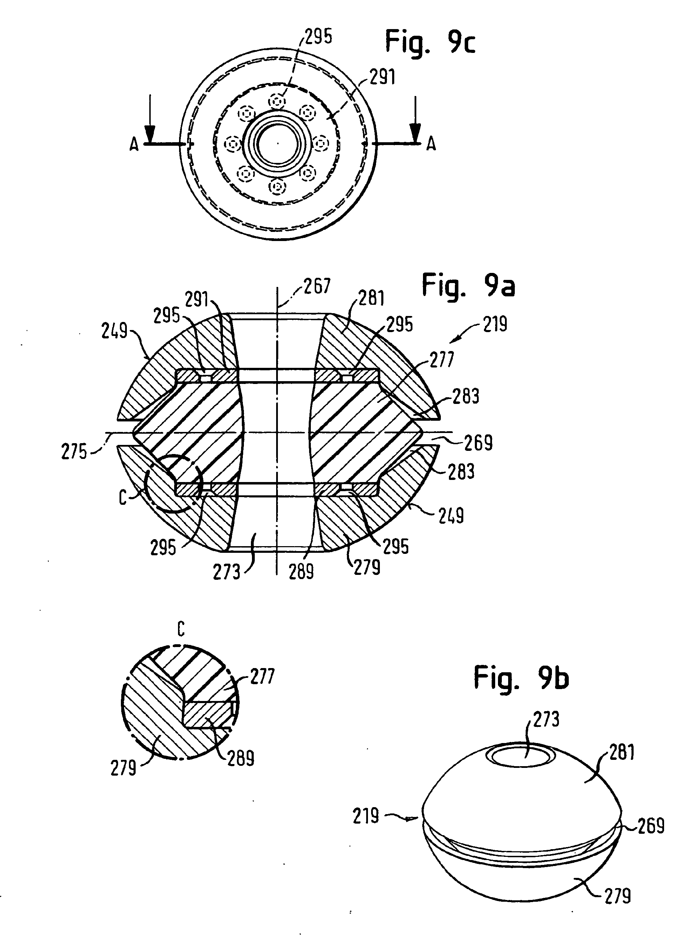

Bei dem in den Fig. 9a - 9c dargestellten erfindungsgemäßen Implantatkern 219 schließt das Stützkissen 277 nach unten und oben jeweils bündig mit einer aus Metall hergestellten, ringförmigen Zwischenlage 289, 291 ab. Der Außendurchmesser der sich parallel zur Äquatorialebene 275 erstreckenden Zwischenringe 289, 291 beträgt etwa 60 % des Außendurchmessers der äußeren PE-Halbschalen 279, 281, während der Innendurchmesser der Zwischenringe 289, 291 etwa 24 % des Halbschalen-Außendurchmessers beträgt.

Jeweils ausgehend von den ringförmigen Zwischenlagen 289, 291 nimmt der Durchmesser des Stützkissens 277 in Richtung der Äquatorialebene 275 zu, wobei jedoch radial außerhalb der Zwischenlagen 289, 291 jeweils ein nach außen hin breiter werdender Zwischenraum 283 zwischen dem Stützkissen 277 und den Halbschalen 279, 281 vorhanden ist. Die Halbschalen 279, 281 sind folglich ausschließlich über die Metallringe 289, 291 am Stützkissen 277 abgestützt.

Das Stützkissen 277 bildet mit den Metall-Zwischenringen 289, 291 einen festen Materialverbund, der durch Anspritzen des für das Stützkissen 277 vorgesehenen Materials, für das z.B. die vorstehend bereits genannten Materialien in Frage kommen, an die Innenseiten der Zwischenlagen 289, 291 hergestellt wird. Eine zusätzliche formschlüssige Verbindung entsteht durch in den Zwischenlagen 289, 291 ausgebildete hinterschnittene Bohrungen 295, in welche das Material des Stützkissens 277 bei der Herstellung hineinfließt. Wie Fig. 9c zeigt, ist eine Vielzahl von kreisförmig mit gleichmäßigem Abstand voneinander angeordneten Hinterschneidungen 295 vorgesehen.

Wie das "Detail C" der Fig. 9a zeigt, sind die Seitenkanten der Zwischenringe 289, 291 sowie die radial äußeren Begrenzungsseiten von in den Halbschalen 279, 281 ausgebildeten Aufnahmebereichen hinterschnitten, so dass zwischen dem Verbund aus Stützkissen 277 und Zwischenringen 289, 291 einerseits und den beiden Halbschalen 279, 281 andererseits jeweils eine Schnappverbindung herstellbar ist. Die Halbschalen 279, 281 können also einfach auf das mit den Metallringen 289, 291 fest verbundene Stützkissen 277 aufgeclipst werden.

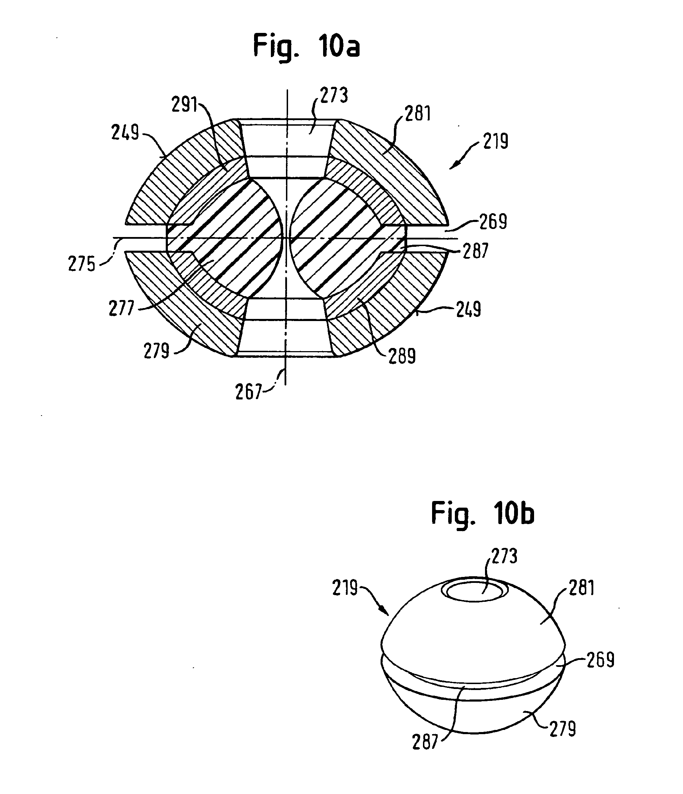

Bei dem Ausführungsbeispiel der Fig. 10a und 10b sind zwischen dem aus PCU hergestellten Stützkissen 277 und den PE-Schalen 279, 281 wiederum ringförmige Zwischenlagen 289, 291 aus Metall angeordnet. In diesem Ausführungsbeispiel verlaufen die Zwischenringe 289, 291 jedoch nicht senkrecht zur Mittelachse 267 des Implantatkerns 219, sondern sind entsprechend den die Artikulationsflächen 249 bereitstellenden äußeren Halbschalen 279, 281 gekrümmt.

Die radiale Innenseite des ringförmigen Stützkissens 277 ist vergleichsweise stark konvex gekrümmt, wobei der zentrale Durchgang 273 in der Äquatorialebene 275 eine ausgeprägte Engstelle aufweist.

In radialer Richtung schließt das Stützkissen 277 über einen zwischen den Metall-Zwischenringen 289, 291 liegenden, flanschartigen Abschnitt 287 bündig mit den Zwischenringen 289, 291 ab. Die PE-Halbschalen 279, 281 besitzen also wiederum einen Überhang bezüglich des Verbundes aus Stützkissen 277 und Zwischenringen 289, 291. Die Halbschalen 279, 281 schließen in axialer Richtung jeweils bündig mit den Zwischenringen 289, 291 ab, wodurch der Implantatkern 219 eine äußere Ringnut 269 aufweist, deren axiale Höhe der Dicke des Flanschabschnitts 287 des Stützkissens 277 entspricht.

Die Verbindung zwischen den aus einer CoCrMo-Legierung hergestellten Zwischenlagen 289, 291 und den PE-Halbschalen 279, 281 erfolgt jeweils durch Anspritzen des PE-Materials an die Außenseiten der Metall-Zwischenlagen 289, 291, die hierzu mit in Fig. 10a nicht dargestellten Rücksprüngen bzw. Hinterschneidungen versehen sind, in welche das PE-Material beim Anspritzen hineinfließen kann. Vorzugsweise sind diese Hinterschneidungen in Form von kreisringförmigen, zurückspringenden Stufen vorgesehen, deren Breite und Höhe mit der radialen Position derart variiert, dass von innen nach außen die Stufenbreite abnimmt und die Stufenhöhe zunimmt. Diese Herstellung des Materialverbundes kann prinzipiell auch bei anderen Materialpaarungen zum Einsatz kommen, ist also nicht auf PE für die Halbschalen und eine CoCrMo-Legierung für die Zwischenlagen beschränkt.

Für die Ausführungsbeispiele der Fig. 9 und 10 ist es bevorzugt, wenn die von den hier nicht dargestellten Implantatplatten vorstehenden, in den zentralen Durchgang 273 hinein ragenden Zapfen (vgl. Fig. 5 und 6) bis zu den metallischen Zwischenlagen 289, 291 reichen, da dann bei aufgrund der Artikulation erfolgenden Kippbewegungen der Implantatplatten relativ zum Implantatkern 219 die Metallringe 289, 291 ohne Beeinträchtigung der PE-Halbschalen 279, 281 als Wegbegrenzung für die Zapfen und damit die Implantatplatten dienen können.

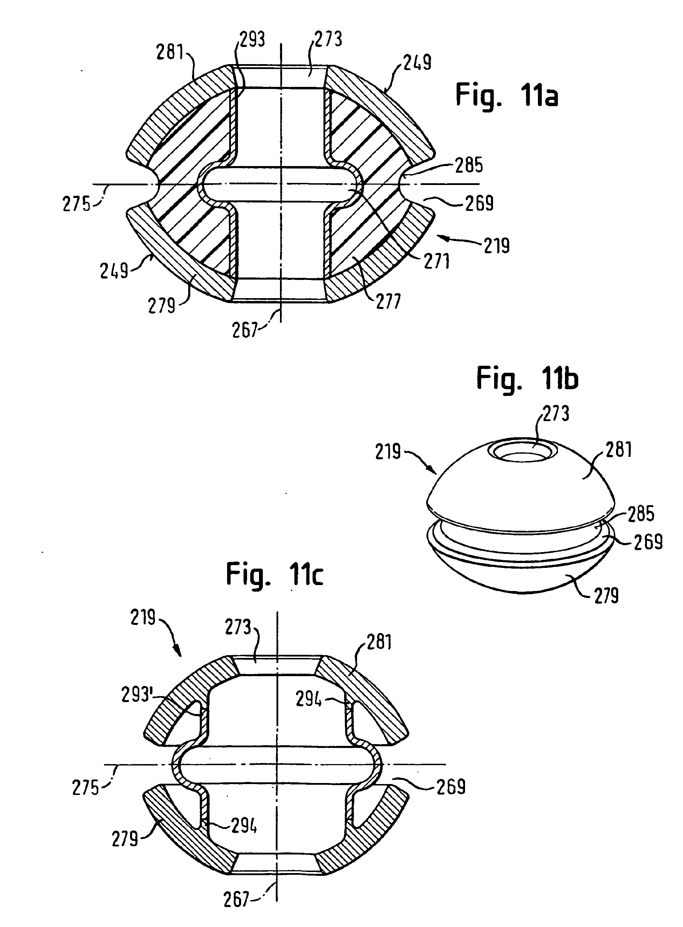

Der Implantatkern 219 gemäß Fig. 11a,11b weist keine Zwischenlagen zwischen dem wiederum aus PCU hergestellten Stützkissen 277 und den äußeren Halbschalen 279, 281 auf, die in diesem Ausführungsbeispiel nicht aus PE, sondern aus Metall hergestellt sind. Die Verbindung zwischen dem PCU-Stützkissen 277 und den Halbschalten 279, 281 erfolgt durch Anspritzen des PCU-Materials.

An seiner radialen Innenseite ist das Stützkissen 277 durch ein als Metallfaltenbalg ausgebildetes Versteifungselement 293 abgestützt, das bis an die Innenseiten der Metall-Halbschalen 279, 281 heranreicht. Zum einen wird hierdurch die Steifigkeit des Stützkissens 277 in axialer Richtung erhöht. Zum anderen ergibt sich durch das Versteifungselement 293 eine verbesserte Führung der Halbschalen 279, 281 relativ zueinander, wodurch verhindert wird, dass die Halbschalen 279, 281 auf dem Stützkissen 277 "schwimmen".

Die Halbschalen 279, 281 und das Versteifungselement 293 sind bevorzugt aus demselben Material hergestellt, für welches insbesondere eine CoCrMo-Legierung verwendet wird.

Die Wandstärke der Halbschalen 279, 281 liegt in der Größenordnung von etwa 1 mm, wodurch sich eine ausreichende Formelastizität ergibt. Eine resultierende Abstützung der Halbschalen 279, 281 im mittleren Bereich zwischen einer radial äußeren Ringnut 285 des Stützkissens 277 und der Innenseite, d.h. dem Versteifungselement 293, lässt eine vergleichsweise geringe Formänderung der Halbschalen 279, 281 im radial äußeren Randbereich in der Größenordnung von µm in axialer Richtung zu.

Die äußere Ringnut 285 des Stützkissens 277 und der Ringspalt zwischen den insofern axial beabstandeten Halbschalen 279, 281 bilden zusammen eine äußere Ringnut 269 des gesamten Implantatkerns 219. Durch eine radial nach außen gerichtete Ausstülpung des Metallfaltenbalgs 293 in Höhe der Äquatorialebene 275 entsteht eine innere Ringnut 271.

Die von Teilkugelflächen gebildeten Artikulationsflächen 249 der in diesem Ausführungsbeispiel aus Metall hergestellten Halbschalen 279, 281 sowie die entsprechenden Artikulationsflächen der nicht dargestellten Implantatplatten lassen sich - wenn die Zapfen der Implantatplatten (vgl. z.B. Fig. 5 und 6) nachträglich an den Implantatplatten z.B. durch Einpressen angebracht werden - mittels des in der vorstehend bereits erwähnten europäischen Patentanmeldung mit der Veröffentlichungsnummer EP 0 892 627 mit derjenigen Genauigkeit bearbeiten, die erforderlich ist, um über die Formelastizität der radial äußeren Randbereiche der Halbschalen 279, 281 die gewünschte Verminderung der Flächenpressung in diesen Randbereichen zu erzielen.

Alternativ zu der in Fig. 11a und 11b dargestellten Ausführungsform kann gemäß einer weiteren, in Fig. 11c dargestellten Variante der Erfindung das Stützkissen auch weggelassen werden und die Abstützung der Halbschalen 279, 281 ausschließlich über ein Versteifungselement 293' z.B. entsprechend dem Faltenbalg 293 gemäß Fig. 11a erfolgen. In dieser Variante ist das Versteifungselement 293' gegenüber der in Fig. 11a gezeigten Position nach radial außen versetzt, d.h. mit einem größeren Durchmesser versehen. Dadurch erfolgt die Abstützung der Halbschalen 279, 281 in einem mittleren Bereich - in welchem die Halbschalen 279, 281 jeweils einen axialen Ringvorsprung für das Versteifungselement 293' aufweisen - zwischen radial äußerem Rand einerseits und den zentralen Durchgang 273 bzw. die zentralen Öffnungen der ringförmigen Halbschalen 279, 281 begrenzendem Innenrand andererseits, wodurch wiederum Druckspitzen in diesen radial äußeren und inneren Randbereichen vermieden werden.

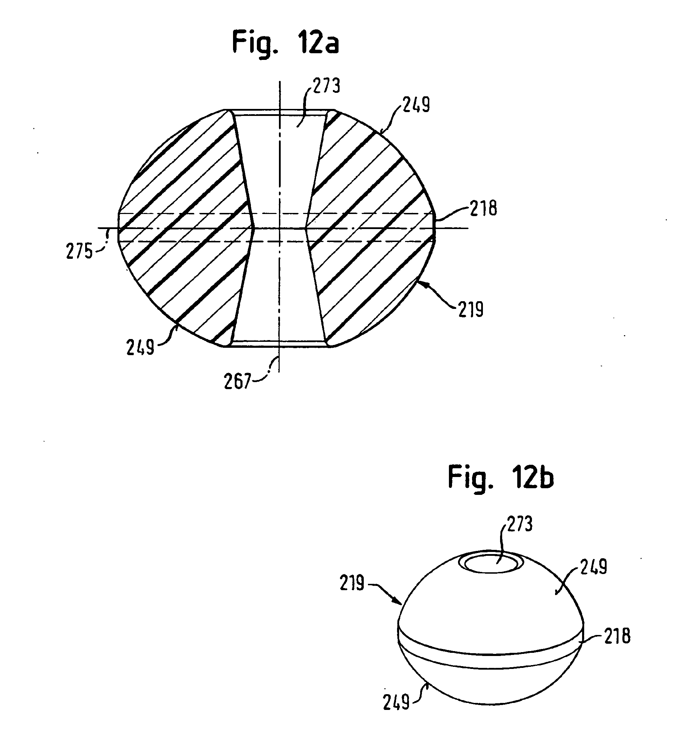

In dem Beispiel der Fig. 12a und 12b wird der Implantatkern 219 ausschließlich von einem PCU-Stützkissen gebildet, das mit einem zur Äquatorialebene 275 symmetrischen zentralen Durchgang 273 in Form eines in der Äquatorialebene 275 zusammenlaufenden Doppelkonus versehen ist.

Der Implantatkern 219 besitzt die Form zweier mit ihren ebenen Seiten einander zugewandter Kugelsegmente und dazwischen liegender Zylinderscheibe 218. Die axiale Höhe dieser Zylinderscheibe 218 ist derart gewählt, dass die nicht dargestellten, mit den Artikulationsflächen 249 des Implantatkerns 219 zusammenwirkenden Teilkugelflächen der Implantatplatten in jeder zulässigen Artikulationsstellung die Zylinderscheibe 218 noch überdecken, d.h. einen ausreichend großen Überstand aufweisen.

Aufgrund der vergleichsweise hohen Elastizität des den Implantatkern 219 bildenden PCU-Materials erfolgt unter Belastung eine Kompression nicht nur in der axialen, sondern auch in der radialen Richtung, wodurch der axial äußere Randbereich der Artikulationsflächen 249 entlastet wird. Auch bei übereinstimmenden Krümmungsradien R0 zwischen Implantatkern 219 und Implantatplatten stellt sich folglich eine Reduzierung der Druckbelastung der Artikulationsflächen 249 in Richtung der axial äußeren Randbereiche ein.

Zur radialen Innenseite hin kann die sich unter Belastung einstellende Druckverteilung durch die Formgebung des hier doppelkonusförmigen zentralen Durchgangs 273 gezielt eingestellt werden.

Die Artikulationsflächen 249 des PCU-Implantatkerns 219 können zusätzlich mit einer Vernetzung und/oder Beschichtung versehen sein, die dazu dient, den Verschleiß zu reduzieren. Die Verschleißreduzierung kann dabei durch eine höhere Festigkeit und/oder durch einen niedrigeren Reibungswert erzielt werden.

Die vorstehend anhand der Fig. 7 bis 12 erläuterten Implantatkerne 219 haben die folgenden Abmessungen, wobei diesbezüglich außerdem auf den Einleitungsteil Bezug verwiesen wird:

Der kleinste Innendurchmesser des ringförmigen Stützkissens 277, d.h. der Durchmesser des zentralen Durchgangs 273 an der engsten, in der Äquatorialebene 275 gelegenen Engstelle beträgt etwa 5mm in den Beispielen der Fig. 7, 8 und 9, etwa 0,4mm im Beispiel der Fig. 10 und etwa 4mm im Beispiel der Fig. 12.

Der größte Durchmesser des zentralen Durchgangs 273 an der Außenseite der Halbschalen 279, 281 bzw. des Implantatkerns 219 beträgt in den Beispielen der Fig. 7 und 8 etwa 7,4mm und in dem Beispiel der Fig. 12 etwa 7,3mm.

Der Abstand zwischen den Mittelpunkten der die Teilkugelflächen 249 definierenden Kugelsegmente beträgt in den Beispielen der Fig. 7, 8 und 11 etwa 6mm und im Beispiel der Fig. 12 etwa 5mm.

Der Öffnungswinkel des zentralen Durchgangs 273 an der Außenseite der Halbschalen 279, 281 beträgt in den Beispielen der Fig. 7 und 8 etwa 20°.

Die axiale Höhe des radial äußeren Ringspaltes zwischen den Halbschalen 279, 281 beträgt in den Beispielen der Fig. 7, 8 und 10 etwa 2mm. Im Beispiel der Fig. 11 beträgt der kleinste Abstand zwischen den Halbschalen 279, 281 und damit die maximale axiale Breite der äußeren Ringnut 285 des Stützkissen 277 (Fig. 11a und 11b) etwa 2,6mm.

Im Beispiel der Fig. 9 beträgt der axiale Abstand zwischen den Metallringen 289, 291, d.h. die axiale Höhe des Stützkissens 277, etwa 8mm und der Durchmesser des zentralen Durchgangs 273 in Höhe der Außenseiten der Metallringe 289, 291 etwa 6mm. Die Dicke der Metallringe 289, 291 beträgt etwa 1mm.

Im Beispiel der Fig. 10 beträgt die Wandstärke der ringförmigen Zwischenlagen 289, 291 etwa 1,7mm. Der Durchmesser des zentralen Durchgangs 273 bei der maximalen axialen Höhe des Stützkissen 277, d.h. der kleinste Durchmesser der Zwischenringe 289, 291, beträgt etwa 6mm.

Im Beispiel der Fig. 11a und 11b beträgt der Innendurchmesser des Versteifungselementes 293 etwa 6,7mm, während dessen Wandstärke - auch im Beispiel der Fig. 11c - etwa 0,5mm beträgt.

### Bezugszeichenliste

- 15, 115: Implantatplatte
- 17, 117: Implantatplatte
- 18, 218: Zwischenscheibe
- 19, 119,219: Implantatkern
- 20: Aussparung für Adapterelement
- 41, 41': dom- bzw. tonnenförmige Erweiterung
- 42: Anschlagstift
- 43, 43': Führungsvorsprung, Finne bzw. Haltevorsprung
- 44: Bohrung
- 45: Vertiefung der Implantatplatte
- 47, 147: Artikulationsfläche der Vertiefung bzw. Implantatplatte
- 49, 149, 249: Artikulationsfläche des Implantatkerns
- 51, 151: Zapfen
- 53: Ausnehmung
- 55: Einführkanal des Implantatkerns
- 57: Einführkanal der Implantatplatte
- 59: Fluidleitung
- 61: Wirbelkörper
- 63: Wölbung
- 65: Randbereich

- M: Kugelmittelpunkt
- R: Radius der Artikulationsflächen
- O: Nullreferenz
- α: Angulation

- H: Höhe der Implantatplatten
- B: Breite der Implantatplatten
- T: Tiefe der Implantatplatten
- d: Domposition
- h: Domhöhe
- z: Wölbungszentrum
- w: Wölbungshöhe
- a: Finnenabstand
- f: Finnenhöhe

- v: Abstand der Aussparungen

- 167, 267: Mittelachse des Implantatkerns
- 169, 269: äußere Ringnut
- 171, 271: innere Ringnut
- 173, 273: zentraler Durchgang
- M0, M1, M2: Mittelpunkt
- R0,R1, R2: Krümmungsradius
- P: Berührungslinie
- t: Tangente

- ω: Winkel

- 275: Äquatorialebene
- 277: Stützkissen
- 279: Halbschale
- 281: Halbschale
- 283: Zwischenraum
- 285: äußere Ringnut des Stützkissens
- 287: Flanschabschnitt es Stützkissens
- 289: Zwischenlage
- 291: Zwischenlage
- 293, 293': Versteifungselement
- 294: Ringvorsprung
- 295: Rücksprung, Hinterschneidung

## Patentansprüche

1. Bandscheibenimplantat mit zwei im implantierten Zustand an vorbereiteten Wirbelkörperflächen anliegenden Implantatplatten und einem zwischen die Implantatplatten einbringbaren Implantatkern (219), wobei der Implantatkern (219) mehrteilig ausgebildet ist und eine Anordnung aus wenigstens einem inneren Stützkissen (277) und zumindest einer das Stützkissen (277) umgebenden Schale (279, 281) umfasst, und
wobei die Schale zwei Halbschalen (279, 281) umfasst, die jeweils in radialer Richtung über das Stützkissen (277) hinaus vorstehen und die in axialer Richtung mit Abstand voneinander angeordnet sind,
**dadurch gekennzeichnet,**
**dass** die Halbschalen (279, 281) jeweils eine konkave, im implantierten Zustand mit dem Stützkissen (277) zusammenwirkende Seite und eine konvexe, im implantierten Zustand mit einer der Implantatplatten zusammenwirkende Seite aufweisen.

2. Bandscheibenimplantat nach Anspruch 1,
**dadurch gekennzeichnet,**
**dass** die Anordnung aus innerem Stützkissen (277) und umgebender Schale (279, 281) als Materialverbund ausgebildet ist.

3. Bandscheibenimplantat nach Anspruch 1 oder 2,
**dadurch gekennzeichnet,**
**dass** das Stützkissen (277) eine linsenförmige Grundform aufweist.

4. Bandscheibenimplantat nach einem der vorhergehenden Ansprüche,
**dadurch gekennzeichnet,**
**dass** das Stützkissen (277) und die Schale (279, 281) aus unterschiedlichen Materialien hergestellt sind, wobei bevorzugt das Material der Schale (279, 281) härter und/oder steifer ist als das Material des Stützkissens (277).

5. Bandscheibenimplantat nach einem der vorhergehenden Ansprüche,
**dadurch gekennzeichnet,**
**dass** in einem radial äußeren Randbereich zwischen der Schale (279, 281) und dem Stützkissen (277) ein Zwischenraum (283) vorhanden ist.

6. Bandscheibenimplantat nach einem der vorhergehenden Ansprüche,
**dadurch gekennzeichnet,**
**dass** zwischen dem Stützkissen (277) und der Schale (279, 281) eine insbesondere aus Metall hergestellte Zwischenlage (289, 291) angeordnet ist.

7. Bandscheibenimplantat nach Anspruch 6,
**dadurch gekennzeichnet,**
**dass** die Zwischenlage (289, 291) als Wegbegrenzung für in einen senkrecht zu einer Äquatorialebene (275) verlaufenden Durchgang (273) hineinragende Zapfen der Implantatplatten ausgebildet ist.

8. Bandscheibenimplantat nach einem der vorhergehenden Ansprüche,
**dadurch gekennzeichnet,**
**dass** ein senkrecht zu einer Äquatorialebene (275) verlaufender Durchgang (273) des Implantatkerns (219) eine über seine Länge variierende Querschnittsfläche aufweist, wobei vorzugsweise die Querschnittsfläche jeweils von der Äquatorialebene (275) nach außen insbesondere stetig zunimmt.

9. Bandscheibenimplantat nach einem der vorhergehenden Ansprüche,
**dadurch gekennzeichnet,**
**dass** ein Durchgang (273) eine von einer Innenwand des Stützkissens (277) begrenzte Engstelle aufweist.

10. Bandscheibenimplantat nach einem der vorhergehenden Ansprüche,
**dadurch gekennzeichnet,**
**dass** das Stützkissen (277) ringförmig ausgebildet ist, wobei bevorzugt die in radialer Richtung gemessene Ringdicke in der Äquatorialebene (275) am größten ist und jeweils mit zunehmender axialer Entfernung von der Äquatorialebene (275) abnimmt.

11. Bandscheibenimplantat nach einem der vorhergehenden Ansprüche,
**dadurch gekennzeichnet,**
**dass** das Stützkissen (277) in einem zentralen Bereich in axialer Richtung versteift ist.

12. Bandscheibenimplantat nach einem der vorhergehenden Ansprüche,
**dadurch gekennzeichnet,**
**dass** das Stützkissen (277) in radialer Richtung nach innen hin versteift ist.

13. Bandscheibenimplantat nach einem der vorhergehenden Ansprüche,
**dadurch gekennzeichnet,**
**dass** ein separates, insbesondere eine ringförmige oder zylindrische Grundform aufweisendes Versteifungselement (293) vorgesehen ist, das bevorzugt in einem senkrecht zu einer Äquatorialebene (275) verlaufenden Durchgang (273) angeordnet ist.

14. Bandscheibenimplantat nach einem der vorhergehenden Ansprüche,
**dadurch gekennzeichnet,**
**dass** das Stützkissen (277) an die Schale (279, 281), insbesondere an zwei die Schale bildende Halbschalen (279, 281), angespritzt ist, wobei bevorzugt zur Bildung eines durch das Anspritzen herstellbaren Materialverbundes zwischen Stützkissen (277) und Schale (279, 281) das Material des Stützkissens (277) einen höheren Schmelzpunkt aufweist als das Material der Schale (279, 281).

15. Bandscheibenimplantat nach einem der vorhergehenden Ansprüche,
**dadurch gekennzeichnet,**
**dass** das Stützkissen (277) an eine insbesondere aus Metall hergestellte Zwischenlage (289, 291) angespritzt ist.

16. Bandscheibenimplantat nach einem der vorhergehenden Ansprüche,
**dadurch gekennzeichnet,**
**dass** das Stützkissen (277) oder eine mit dem Stützkissen (277) verbundene, insbesondere aus Metall hergestellte Zwischenlage (289, 291) mit der Schale (279, 281), insbesondere mit zwei die Schale bildenden Halbschalen (279, 281), durch eine Clips-, Schnapp- oder Rastverbindung verbunden ist.

17. Bandscheibenimplantat nach einem der vorhergehenden Ansprüche,
**dadurch gekennzeichnet,**
**dass** der Implantatkern (19; 119; 219) eine linsenartige Grundform aufweist.

18. Bandscheibenimplantat nach einem der vorhergehenden Ansprüche,
**dadurch gekennzeichnet,**
**dass** der Implantatkern (19; 119; 219) zumindest näherungsweise die Form zweier mit ihren ebenen Seiten aufeinander liegenden Kugelsegmenten aufweist, wobei jeweils der Kugelmittelpunkt (11) des einen Kugelsegmentes innerhalb des anderen Kugelsegmentes liegt.

19. Bandscheibenimplantat nach einem der Ansprüche 1 bis 17,
**dadurch gekennzeichnet,**
**dass** der Implantatkern (19; 119; 219) zumindest näherungsweise die Form zweier mit ihren ebenen Seiten einander zugewandten Kugelsegmenten und dazwischen liegender Zylinderscheibe aufweist, wobei jeweils der Kugelmittelpunkt (11) des einen Kugelsegmentes innerhalb des anderen Kugelsegmentes liegt.

20. Bandscheibenimplantat nach einem der vorhergehenden Ansprüche,
**dadurch gekennzeichnet,**
**dass** Artikulationsilächen (147) der Implantatplatten (115, 117) jeweils eine Kugelteilfläche mit einem konstanten Krümmungsradius R0 sind und Artikulationsflächen (149) des Implantatkerns (119) jeweils von mehreren unterschiedliche Krümmungsradien (R1, R2) aufweisenden Kugelteilflächen gebildet sind.

21. Bandscheibenimplantat nach einem der vorhergehenden Ansprüche,
**dadurch gekennzeichnet,**
**dass** der Implantatkern (119) bezüglich einer Mittelachse (167) rotationssymmetrisch ausgebildet ist und jede Artikulationsfläche (149) des Implantatkerns (119) in einem Querschnitt durch die Mittelachse (167) einen Krümmungsradius (R1; R1, R2) mit einem Mittelpunkt (M1; M1, M2) aufweist, der kleiner ist als der Krümmungsradius (R0) der Artikulationsflächen (147) der Implantatplatten (115, 117), und
**dass** der Mittelpunkt (M1; M1, M2) der Artikulationsfläche (149) des Implantatkerns (119) von der Mittelachse (167) beabstandet ist.

22. Bandscheibenimplantat nach Anspruch 21,
**dadurch gekennzeichnet,**
**dass** ausgehend von einem Implantatradius, der dem Krümmungsradius (R0) der Artikulationsflächen (147) der Implantatplatten (115, 117) entspricht und von einem auf der Mittelachse (167) des Implantatkerns (119) liegenden Mittelpunkt (M0) aus gemessen wird, die Artikulationsflächen (149) des Implantatkerns (119) einen Krümmungsradius R1<R0 in Richtung des Kernäquators und einen Krümmungsradius R2<R0 in Richtung des Kernpols aufweisen, wobei vorzugsweise R 1 ungleich R2 ist.

23. Bandscheibenimplantat nach Anspruch 22,
**dadurch gekennzeichet,**
dass die Mittelpunkte M0, M 1 und M2 der Krümmungsradien R0, R 1 und R2 auf einer gemeinsamen, bei zusammengesetztem Bandscheibenimplantat die Berührungslinie P zwischen Implantatkern (119) und Implantatplatten (115, 117) schneidenden Geraden liegen, die mit der Äquatorialebene des Implantatkerns (119) einen Winkel ω im Bereich von etwa 45° bis 75°, insbesondere von zumindest näherungsweise 60° einschließt.

24. Bandscheibenimplantat nach einem der Ansprüche 20 bis 23,
**dadurch gekennzeichnet,**
**dass** R0-6mm < R1 < R0-1mm und/oder R2 ≤ R1 gilt, wobei R0 der Krümmungsradius der Artikulationsflächen (147) der Implantatplatten (115, 117) ist.

25. Bandscheibenimplantat nach einem der vorhergehenden Ansprüche,
**dadurch gekennzeichnet,**
**dass** 8mm < R0 < 18mm gilt, wobei R0 der Krümmungsradius der Artikulationsflächen (147) der Implantatplatten (115, 117) ist.

26. Bandscheibenimplantat nach einem der vorhergehenden Ansprüche,
**dadurch gekennzeichnet,**
**dass** der Implantatkern (119; 219) insbesondere im Bereich seiner Äquatorialebene mit einer äußeren Ringnut (169; 269) und/ oder mit einer inneren, bevorzugt eine radiale Erweiterung eines senkrecht zur Äquatorialebene verlaufenden Durchgangs (173; 273) bildenden Ringnut (171; 271) versehen ist.

27. Bandscheibenimplantat nach einem der vorhergehenden Ansprüche,
**dadurch gekennzeichnet,**
**dass** der Implantatkern (119; 219) einen senkrecht zur Äquatorialebene verlaufenden Durchgang (173, 273) aufweist, dessen Längsachse bevorzugt mit der Mittelachse (167; 267) des Implantatkerns (119) zusammenfällt.

28. Bandscheibenimplantat nach einem der vorhergehenden Ansprüche,
**dadurch gekennzeichnet,**
**dass** die Implantatplatten (15, 17) jeweils auf ihrer Außenseite eine domförmige Erweiterung (41) insbesondere in Form eines Kugelsegmentes oder eine tonnenförmige Erweiterung (41') aufweisen.

29. Bandscheibenimplantat nach einem der vorhergehenden Ansprüche,
**dadurch gekennzeichnet,**
**dass** die Außenseiten der Implantatplatten (15, 17) jeweils nach au-ßen gewölbt sind, wobei vorzugsweise die Wölbungen (63) zusätzlich zu dom- oder tonnenförmigen Erweiterungen (41; 41') vorgesehen sind.

30. Bandscheibenimplantat nach einem der vorhergehenden Ansprüche,
**dadurch gekennzeichnet,**
**dass** die Außenseiten der Implantatplatten (15, 17) jeweils einen sich zumindest über einen Teil des Umfangs der Implantatplatten (15, 17) erstreckenden ebenen Randbereich (65) aufweisen.

31. Bandscheibenimplantat nach einem der vorhergehenden Ansprüche,
**dadurch gekennzeichnet,**
**dass** die Implantatplatten (15, 17) jeweils auf ihrer Außenseite wenigstens einen insbesondere als Finne ausgebildeten Führungsvorsprung (43) oder/oder einen insbesondere pyramidenförmigen Haltevorsprung (43') aufweisen.

32. Bandscheibenimplantat nach einem der vorhergehenden Ansprüche,
**dadurch gekennzeichnet,**
**dass** die Implantatplatten (15, 17; 115, 117) jeweils auf ihrer Innenseite eine Vertiefung (45) zur Aufnahme des Implantatkerns (19; 119, 219) aufweisen, wobei vorzugsweise die zusammenwirkenden Artikulationsflächen (47, 49; 147, 149; 249) der Vertiefung (45) und des Implantatkerns (19; 119; 219) jeweils Kugelteilflächen sind.

33. Bandscheibenimplantat nach einem der vorhergehenden Ansprüche,
**dadurch gekennzeichnet,**
**dass** zumindest eine Implantatplatte (15, 17; 115, 117) einen von ihrer Innenseite abstehenden Zapfen (51; 151) aufweist, der bei zusammengesetztem Implantat in eine auf der Außenseite des Implantatkerns (19; 119; 219) ausgebildete Ausnehmung (53) hineinragt, wobei die Ausnehmung (53) größer als der Zapfen (51; 151) bemessen ist, um eine Relativbewegung zwischen Implantatplatte (15, 17; 115, 117) und Implantatkern (19; 119; 219) zu ermöglichen.

34. Bandscheibenimplantat nach Anspruch 33,
**dadurch gekennzeichnet,**
**dass** der Zapfen (51; 151) mittig bezüglich der Abmessung der Implantatplatte in sagittaler Richtung angeordnet ist.

35. Bandscheibenimplantat nach Anspruch 33,
**dadurch gekennzeichnet,**
**dass** der Zapfen (51; 151) exzentrisch bezüglich der Abmessung der Implantatplatte in sagittaler Richtung angeordnet ist.

36. Bandscheibenimplantat nach einem der vorhergehenden Ansprüche,
**dadurch gekennzeichnet,**
**dass** der Implantatkern (19) auf zumindest einer Außenseite mit einem vom Rand zur Ausnehmung (53) verlaufenden Einführkanal (55) für den Zapfen (51) der Implantatplatte (15, 17) versehen ist.

37. Bandscheibenimplantat nach einem der vorhergehenden Ansprüche,
**dadurch gekennzeichnet,**
**dass** zumindest eine Implantatplatte (15, 17) auf ihrer Innenseite mit einem vom Rand zur Vertiefung (45) verlaufenden Einführkanal (57) für den Implantatkern (19) versehen ist.

38. Verfahren zur Herstellung eines Bandscheibenimplantats nach Anspruch 1, wobei das Stützkissen (277) in einem Kunststoffspritzverfahren an die Schale (279, 281), insbesondere an die Halbschalen, oder an eine im fertigen Zustand zwischen dem Stützkissen (277) und der Schale (279, 281) angeordnete Zwischenlage (289, 291) angespritzt wird.

39. Verfahren nach Anspruch 38,
**dadurch gekennzeichnet,**
**dass** zur Herstellung eines Materialverbundes zwischen Stützkissen (277) und Schale (279, 281) ein Material für das anzuspritzende Stützkissen (277) gewählt wird, das einen höheren Schmelzpunkt aufweist als das Material der Schale (279, 281).

40. Verfahren nach Anspruch 38 oder 39,
**dadurch gekennzeichnet,**
**dass** die Zwischenlage (289, 291) aus Metall hergestellt wird.

41. Verfahren nach einem der Ansprüche 38 bis 40,
**dadurch gekennzeichnet,**
**dass** beim Spritzen des Stützkissens (277) an der Innenseite der Schale (279, 281) bzw. der Zwischenlage (289, 291) ausgebildete Rücksprünge oder Hinterschneidungen (295) ausgespritzt werden.

## Claims

1. An intervertebral disk implant having two implant plates contacting prepared vertebral body surfaces in the implanted state and an implant core (219) which can be introduced between the implant plates, wherein the implant core (219) is made in multiple parts and includes an arrangement of at least one inner support cushion (277) and at least one shell (279, 281) surrounding the support cushion (277); and
wherein the shell includes two half shells (279, 281) which each project beyond the support cushion (277) in the radial direction and are arranged spaced apart from one another in the axial direction, **characterized in that**
the half shells (279, 281) each have a concave side cooperating with the support cushion (277) in the implanted state and a convex side cooperating with one of the implant plates in the implanted state.

2. An intervertebral disk implant in accordance with claim 1, **characterized in that** the arrangement of inner support cushion (277) and surrounding shell (279, 281) is made as a material composite.

3. An intervertebral disk implant in accordance with claim 1 or claim 2, **characterized in that** the support cushion (277) has a lens-like base shape.

4. An intervertebral disk implant in accordance with any one of the preceding claims, **characterized in that** the support cushion (277) and the shell (279, 281) are made from different materials, with the material of the shell (279, 281) preferably being harder and/or stiffer than the material of the support cushion (277).

5. An intervertebral disk implant in accordance with any one of the preceding claims, **characterized in that** an intermediate space (283) is present in a radially outer rim region between the shell (279, 281) and the support cushion (277).

6. An intervertebral disk implant in accordance with any one of the preceding claims, **characterized in that** an intermediate layer (289, 291), in particular made of metal, is arranged between the support cushion (277) and the shell (279, 281).

7. An intervertebral disk implant in accordance with claim 6, **characterized in that** the intermediate layer (289, 291) is made as a path boundary for spigots of the implant plates projecting into a passage (273) extending perpendicular to an equatorial plane (275).

8. An intervertebral disk implant in accordance with any one of the preceding claims, **characterized in that** a passage (273) of the implant core (219) extending perpendicular to an equatorial plane (275) has a cross-sectional area which varies over its length, with the cross-sectional area preferably respectively increasing, in particular constantly, outwardly from the equatorial plane (275).

9. An intervertebral disk implant in accordance with any one of the preceding claims, **characterized in that** a passage (273) has a restriction bounded by an inner wall of the support cushion (277).

10. An intervertebral disk implant in accordance with any one of the preceding claims, **characterized in that** the support cushion (277) is made in ring shape, with the ring thickness measured in the radial direction preferably being the largest in the equatorial plane (275) and in each case reducing with increasing axial distance from the equatorial plane (275).

11. An intervertebral disk implant in accordance with any one of the preceding claims, **characterized in that** the support cushion (277) is stiffened in a central region in the axial direction.

12. An intervertebral disk implant in accordance with any one of the preceding claims, **characterized in that** the support cushion (277) is stiffened inwardly in the radial direction.

13. An intervertebral disk implant in accordance with any one of the preceding claims, **characterized in that** a separate stiffening element (293), in particular having a ring shaped or cylindrical base shape, is provided which is preferably arranged in a passage (273) extending perpendicular to an equatorial plane (275).

14. An intervertebral disk implant in accordance with any one of the preceding claims, **characterized in that** the support cushion (277) is injection molded onto the shell (279, 281), in particular onto two half shells (279, 281) forming the shell, with the material of the support cushion (277) preferably having a higher melting point than the material of the shell (279, 281) for the forming of a material composite between the support cushion (277) and the shell (279, 281) which can be manufactured by injection molding.

15. An intervertebral disk implant in accordance with any one of the preceding claims, **characterized in that** the support cushion (277) is injection molded onto an intermediate layer (289, 291), in particular made of metal.

16. An intervertebral disk implant in accordance with any one of the preceding claims, **characterized in that** the support cushion (277) or an intermediate layer (289, 291) connected to the support cushion (277) and in particular made of metal is connected to the shell (279, 281), in particular to two half shells (279, 281) forming the shell, by a clip, snap, or latch connection.

17. An intervertebral disk implant in accordance with any one of the preceding claims, **characterized in that** the implant core (19; 119; 219) has a lens-like basic shape.

18. An intervertebral disk implant in accordance with any one of the preceding claims, **characterized in that** the implant core (19; 119; 219) has at least approximately the shape of two spherical segments whose planar sides lie on top of one another, with the respective spherical center (11) of the one spherical segment lying within the other spherical segment.

19. An intervertebral disk implant in accordance with any one of the claims 1 to 17, **characterized in that** the implant core (19; 119; 219) has at least approximately the shape of two spherical segments whose planar sides face one another and a cylindrical disk disposed therebetween, with the respective spherical center (11) of the one spherical segment lying within the other spherical segment.

20. An intervertebral disk implant in accordance with any one of the preceding claims, **characterized in that** articulation surfaces (147) of the implant plates (115, 117) are each a part surface of a sphere having a constant radius of curvature R0 and articulation surfaces (149) of the implant core (119) are each formed by a plurality of part surfaces of a sphere having different radii of curvature (R1, R2).

21. An intervertebral disk implant in accordance with any one of the preceding claims, **characterized in that** the implant core (119) is made rotationally symmetrical with respect to a central axis (167) and each articulation surface (149) of the implant core (119) has a radius of curvature (R1; R1, R2) in a cross-section through the central axis (167), said radius of curvature having a center (M 1; M 1, M2) being smaller than the radius of curvature (R0) of the articulation surfaces (147) of the implant plates (115, 117); and **in that** the center (M1; M1, M2) of the articulation surface (149) of the implant core (119) is spaced apart from the central axis (167).

22. An intervertebral disk implant in accordance with claim 21, **characterized in that**, starting from an implant radius which corresponds to the radius of curvature (R0) of the articulation surfaces (147) of the implant plates (115, 117) and is measured from a center (M0) disposed on the central axis (167) of the implant core (119), the articulation surfaces (149) of the implant core (119) have a radius of curvature R1<R0 in the direction of the core equator and a radius of curvature R2<R0 in the direction of the core pole, with R1 preferably not being equal to R2.

23. An intervertebral disk implant in accordance with claim 22, **characterized in that** the centers M0, M1 and M2 of the radii of curvature R0, R 1 and R2 lie on a common straight line which intersects the contact line P between the implant core (119) and the implant plates (115, 117), when the intervertebral disk implant is assembled, and which includes an angle ω with the equatorial plane of the implant core (119) in the range from approximately 45° to 75°, in particular from at least approximately 60°.

24. An intervertebral disk implant in accordance with any one of the claims 20 to 23, **characterized in that** R0-6 mm < R1 < R0-1 mm and/or R2 ≤ R1 applies, where R0 is the radius of curvature of the articulation surfaces (147) of the implant plates (115, 117).

25. An intervertebral disk implant in accordance with any one of the preceding claims, **characterized in that** 8 mm < R0 < 18 mm applies, where R0 is the radius of curvature of the articulation surfaces (147) of the implant plates (115, 117) .

26. An intervertebral disk implant in accordance with any one of the preceding claims, **characterized in that** the implant core (119; 219) is provided, in particular in the region of its equatorial plane, with an outer ring groove (169; 269) and/or with an inner ring groove (171; 271) preferably forming a radial extension of a passage (173; 273) extending perpendicular to the equatorial plane.

27. An intervertebral disk implant in accordance with any one of the preceding claims, **characterized in that** the implant core (119; 219) has a passage (173; 273) extending perpendicular to the equatorial plane whose longitudinal axis preferably coincides with the central axis (167; 267) of the implant core (119).

28. An intervertebral disk implant in accordance with any one of the preceding claims, **characterized in that** the implant plates (15, 17) each have a dome-shaped extension (41), in particular in the form of a spherical segment, or a barrel-shaped extension (41') at their outer sides.

29. An intervertebral disk implant in accordance with any one of the preceding claims, **characterized in that** the outer sides of the implant plates (15, 17) are each outwardly arched, with the arches (63) preferably being provided in addition to a dome-shaped extension or a barrel-shaped extension (41; 41').

30. An intervertebral disk implant in accordance with any one of the preceding claims, **characterized in that** the outer sides of the implant plates (15, 17) each have a planar rim region (65) extending at least over part of the periphery of the implant plates (15, 17).

31. An intervertebral disk implant in accordance with any one of the preceding claims, **characterized in that** the implant plates (15, 17) each have at least one guide projection (43), in particular formed as a fin, and/or a holding projection (43'), in particular a pyramidshaped holding projection, on their outer sides.

32. An intervertebral disk implant in accordance with any one of the preceding claims, **characterized in that** the implant plates (15, 17; 115, 117) each have a recess (45) on their inner sides for the reception of the implant core (19; 119; 219), with the cooperating articulation surfaces (47, 49; 147, 149; 249) of the recess (45) and of the implant core (19; 119; 219) preferably each being part surfaces of a sphere.

33. An intervertebral disk implant in accordance with any one of the preceding claims, **characterized in that** at least one implant plate (15, 17; 115, 117) has a spigot (51; 151) which protrudes from its inner side and which projects into a depression (53) formed on the outer side of the implant core (19; 119; 219), when the implant is assembled, with the depression (53) being dimensioned larger than the spigot (51; 151) in order to permit a relative movement between the implant plate (15, 17; 115; 117) and the implant core (19; 119; 219).

34. An intervertebral disk implant in accordance with claim 33, **characterized in that** the spigot (51; 151) is arranged centrally with respect to the dimension of the implant plate in the sagittal direction.

35. An intervertebral disk implant in accordance with claim 33, **characterized in that** the spigot (51; 151) is arranged eccentrically with respect to the dimension of the implant plate in the sagittal direction.

36. An intervertebral disk implant in accordance with any one of the preceding claims, **characterized in that** the implant core (19) is provided on at least one outer side with an introduction passage (55) for the spigot (51) of the implant plate (15, 17) extending from the rim to the depression (53).

37. An intervertebral disk implant in accordance with any one of the preceding claims **characterized in that** at least one implant plate (15, 17) is provided on its inner side with an introduction passage (57) for the implant core (19) extending from the rim to the recess (45).

38. A method for the manufacture of an intervertebral disk implant in accordance with claim 1, wherein the support cushion (277) is injection molded in a plastic injection method onto the shell (279, 281), in particular onto the half shells, or onto an intermediate layer (289, 291) arranged between the support cushion (277) and the shell (279, 281) in the finished state.

39. A method in accordance with claim 38, **characterized in that** a material for the support cushion (277) to be injection molded is selected for the manufacture of a material composite between the support cushion (277) and the shell (279, 281) which has a higher melting point than the material of the shell (279, 281).

40. A method in accordance with claim 38 or claim 39, **characterized in that** the intermediate layer (289, 291) is made from metal.

41. A method in accordance with any one of the claims 38 to 40, **characterized in that** recesses or undercuts (295) formed at the inner side of the shell (279, 281) or the intermediate layer (289, 291) are injection molded on the injection of the support cushion (277).

## Revendications

1. Implant de disque intervertébral comprenant deux plaques d'implants appliquées, à l'état implanté, contre des surfaces préparées de corps vertébraux et un noyau d'implant (219) susceptible d'être introduit entre les plaques d'implants, et dans lequel le noyau d'implant (219) est réalisé en plusieurs parties et comprend un agencement composé d'au moins un coussin de soutien intérieur (277) et d'au moins une coque (279, 281) qui entoure le coussin de soutien (277), et
dans lequel la coque comprend deux demi-coques (279, 281) qui dépassent respectivement en direction radiale au-delà du coussin de soutien (277) et qui sont agencées à distance l'une de l'autre en direction axiale,
**caractérisé en ce que** les demi-coques (279, 281) présentent chacune un côté concave qui coopère avec le coussin de soutien (277) à l'état implanté, et un côté convexe qui coopère avec l'une des plaques d'implants à l'état implanté.

2. Implant de disque intervertébral selon la revendication 1,
**caractérisé en ce que** l'agencement composé du coussin de soutien intérieur (277) et de la coque (279, 281) qui l'entoure est réalisé sous forme de matériau composite.

3. Implant de disque intervertébral selon la revendication 1 ou 2,
**caractérisé en ce que** le coussin de soutien (277) présente une forme de base semblable à une lentille.

4. Implant de disque intervertébral selon l'une des revendications précédentes,
**caractérisé en ce que** le coussin de soutien (277) et la coque (279, 281) sont fabriqués en matériaux différents, le matériau de la coque (279, 281) étant de préférence plus dur et/ou plus rigide que le matériau du coussin de soutien (277).

5. Implant de disque intervertébral selon l'une des revendications précédentes,
**caractérisé en ce qu'**un espace intermédiaire (283) est prévu dans une région de bordure radiale extérieure entre la coque (279, 281) et le coussin de soutien (277).

6. Implant de disque intervertébral selon l'une des revendications précédentes,
**caractérisé en ce qu'**une couche intermédiaire (289, 291), réalisée en particulier en métal, est agencée entre le coussin de soutien (277) et la coque (279, 281).

7. Implant de disque intervertébral selon la revendication 6,
**caractérisé en ce que** la couche intermédiaire (289, 291) est réalisée à titre de limitation de course pour un tenon, qui pénètre dans une traversée (273) s'étendant perpendiculairement à un plan équatorial (275), des plaques d'implant.

8. Implant de disque intervertébral selon l'une des revendications précédentes,
**caractérisé en ce qu'**une traversée (273), qui s'étend perpendiculairement à un plan équatorial (275), du noyau d'implant (219) présente une surface de section qui varie sur sa longueur, ladite surface de section augmentant de préférence depuis le plan équatorial (275) vers l'extérieur, en particulier en continu.

9. Implant de disque intervertébral selon l'une des revendications précédentes,
**caractérisé en ce qu'**une traversée (273) présente un emplacement rétréci délimité par une paroi intérieure du coussin de soutien (277).

10. Implant de disque intervertébral selon l'une des revendications précédentes,
**caractérisé en ce que** le coussin de soutien (277) est réalisé sous forme annulaire, et l'épaisseur de l'anneau, mesurée en direction radiale, est de préférence maximum dans le plan équatorial (275) et diminue respectivement lorsque l'éloignement axial du plan équatorial (275) augmentent.

11. Implant de disque intervertébral selon l'une des revendications précédentes,
**caractérisé en ce que** le coussin de soutien (277) est rigidifié en direction axiale dans une zone centrale.

12. Implant de disque intervertébral selon l'une des revendications précédentes,
**caractérisé en ce que** le coussin de soutien (277) est rigidifié en direction radiale vers l'intérieur.

13. Implant de disque intervertébral selon l'une des revendications précédentes,
**caractérisé en ce qu'**il est prévu un élément de rigidification séparé (293), présentant en particulier une forme de base annulaire ou cylindrique, qui est de préférence agencé dans une traversée (273) s'étendant perpendiculairement à un plan équatorial (275).

14. Implant de disque intervertébral selon l'une des revendications précédentes,
**caractérisé en ce que** le coussin de soutien (277) est appliqué par injection contre la coque (279, 281), en particulier sur deux demi-coques (279, 281) qui forment la coque et, pour réaliser un matériau composite à réaliser par injection entre le coussin de soutien (277) et la coque (279, 281), le matériau du coussin de soutien (277) présente de préférence une température de fusion plus élevée que le matériau de la coque (279, 281).

15. Implant de disque intervertébral selon l'une des revendications précédentes,
**caractérisé en ce que** le coussin de soutien (277) est appliqué par injection sur une couche intermédiaire (289, 291) réalisée en particulier en métal.

16. Implant de disque intervertébral selon l'une des revendications précédentes,
**caractérisé en ce que** le coussin de soutien (277) ou une couche intermédiaire (289, 291) reliée au coussin de soutien (277), réalisée en particulier en métal, est relié à la coque (279, 281), en particulier à deux demi-coques (279, 281) qui forment la coque, par une liaison à clipsage, à encliquetage ou à enclenchement.

17. Implant de disque intervertébral selon l'une des revendications précédentes,
**caractérisé en ce que** le noyau d'implant (19 ; 119 ; 219) présente une forme de base semblable à une lentille.

18. Implant de disque intervertébral selon l'une des revendications précédentes,
**caractérisé en ce que** le noyau d'implant (19 ; 119 ; 219) présente au moins approximativement la forme de deux segments de sphère posés l'un sur l'autre par leurs côtés plans, le centre respectif (11) de l'un des segments de sphère étant situé à l'intérieur de l'autre segment de sphère.

19. Implant de disque intervertébral selon l'une des revendications 1 à 17,
**caractérisé en ce que** le noyau d'implant (19 ; 119 ; 219) présente au moins approximativement la forme de deux segments de sphère tournés l'un vers l'autre par leurs côtés plans, et d'un disque cylindrique posé entre lesdits segments, le centre respectif (11) de l'un des segments de sphère étant situé à l'intérieur de l'autre segment de sphère.

20. Implant de disque intervertébral selon l'une des revendications précédentes,
**caractérisé en ce que** les surfaces d'articulation (147) des plaques d'implant (115, 117) sont respectivement une surface partielle sphérique avec un rayon de courbure constante R0, et **en ce que** des surfaces d'articulation (149) du noyau d'implant (119) sont formées respectivement par plusieurs surfaces sphériques partielles présentant des rayons de courbure différents (R1, R2).

21. Implant de disque intervertébral selon l'une des revendications précédentes,
**caractérisé en ce que** le noyau d'implant (119) est réalisé à symétrie de révolution par rapport à un axe médian (167), et chaque surface d'articulation (149) du noyau d'implant (119), dans une section prise à travers l'axe médian (167), présente un rayon de courbure (R1 ; R1, R2) avec un centre (M1 ; M1, M2) qui est plus petit que le rayon de courbure (R0) des surfaces d'articulation (147) des plaques d'implant (115, 117), et
**en ce que** le centre (M1 ; M1, M2) des surfaces d'articulation (149) du noyau d'implant (119) est à distance de l'axe médian (167).

22. Implant de disque intervertébral selon la revendication 21,
**caractérisé en ce que**, partant d'un rayon d'implant qui correspond au rayon de courbure (R0) des surfaces d'articulation (147) des plaques d'implant (115, 117) et qui est mesuré depuis un centre (M0) situé sur l'axe médian (167) du noyau d'implant (119), les surfaces d'articulation (149) du noyau d'implant (119) présentent un rayon de courbure R1 < R0 en direction de l'équateur du noyau, et un rayon de courbure R2 < R0 en direction du pôle du noyau, R1 étant de préférence différent de R2.

23. Implant de disque intervertébral selon la revendication 22,
**caractérisé en ce que** les centres M0, M1 et M2 des rayons de courbure R0, R1 et R2, sont situés sur une droite commune qui, lorsque l'implant de disque intervertébral est assemblé, recoupe la ligne de contact P entre le noyau d'implant (119) et les plaques d'implant (115, 117), ladite droite formant, avec le plan équatorial du noyau d'implant (119), un angle γ dans la plage d'environ 45° à 75°, en particulier d'au moins approximativement 60°.

24. Implant de disque intervertébral selon l'une des revendications 20 à 23, **caractérisé en ce que** R0 - 6 mm < R1 < R0 - 1 mm, où R2 ≤ R1 s'applique, R0 étant le rayon de courbure des surfaces d'articulation (147) des plaques d'implant (115, 117).

25. Implant de disque intervertébral selon l'une des revendications précédentes,
**caractérisé en ce que** 8 mm < R0 < 18 mm, R0 étant le rayon de courbure des surfaces d'articulation (147) des plaques d'implant (115, 117).

26. Implant de disque intervertébral selon l'une des revendications précédentes,
**caractérisé en ce que** le noyau d'implant (119 ; 219), en particulier dans la zone de son plan équatorial, est doté d'une gorge annulaire extérieure (169 ; 260) et/ou d'une gorge annulaire (171 ; 271) intérieure formant de préférence un élargissement radial d'une traversée (173 ; 273) s'étendant perpendiculairement au plan équatorial.

27. Implant de disque intervertébral selon l'une des revendications précédentes,
**caractérisé en ce que** le noyau d'implant (119 ; 219) présente une traversée (173, 273) s'étendant perpendiculairement au plan équatorial, dont l'axe longitudinal coïncide de préférence avec l'axe médian (167 ; 267) du noyau d'implant (119).

28. Implant de disque intervertébral selon l'une des revendications précédentes,
**caractérisé en ce que** les plaques d'implant (15, 17) présentent chacune sur leur face extérieure un élargissement (41) en forme de coupole (41) en particulier sous la forme d'un segment de sphère ou un élargissement (41') en forme de tonneau.

29. Implant de disque intervertébral selon l'une des revendications précédentes,
**caractérisé en ce que** les faces extérieures des plaques d'implant (15, 17) sont chacune bombée vers l'extérieur, les bombements (63) étant de préférence prévus additionnellement aux élargissements en forme de coupole ou de tonneau (41 ; 41').

30. Implant de disque intervertébral selon l'une des revendications précédentes,
**caractérisé en ce que** les faces extérieures des plaques d'implant (15, 17) présentent chacune une zone de bordure plane (65) qui s'étend au moins sur une partie de la périphérie des plaques d'implant (15,17).

31. Implant de disque intervertébral selon l'une des revendications précédentes,
**caractérisé en ce que** les plaques d'implant (15, 17) présentent chacune sur leur face extérieure au moins une saillie de guidage (43) réalisé en particulier sous forme d'ailette et/ou une saillie de maintien (43') en particulier de forme pyramidale.

32. Implant de disque intervertébral selon l'une des revendications précédentes,
**caractérisé en ce que** les plaques d'implant (15, 17 ; 115, 17) présentent chacune sur leur face intérieure un renfoncement (45) pour la réception du noyau d'implant (19 ; 119, 219), les surfaces d'articulation coopérantes (47, 49 ; 147,149 ; 249) du renfoncement (45) et du noyau d'implant (19 ; 119 ; 219) étant de préférence des surfaces partiellement sphériques.

33. Implant de disque intervertébral selon l'une des revendications précédentes,
**caractérisé en ce qu'**au moins une plaque d'implant (15, 17 ; 115, 117) présente un tenon (51 ; 151) qui dépasse de sa face intérieure et qui, lorsque l'implant est assemblé, fait saillie dans un évidement (53) réalisé sur la face extérieure du noyau d'implant (19 ; 119 ; 219), ledit évidement (53) étant de dimension supérieure au tenon (51 ; 151) pour permettre un mouvement relatif entre la plaque d'implant (15, 17 ; 115, 17) et le noyau d'implant (19 ; 119 ; 219).

34. Implant de disque intervertébral selon la revendication 33,
**caractérisé en ce que** le tenon (51 ; 151) est agencé au milieu, en direction sagittale, par référence à la dimension de la plaque d'implant.

35. Implant de disque intervertébral selon la revendication 33,
**caractérisé en ce que** le tenon (51 ; 151) est agencé de façon excentrique, en direction sagittale, par référence à la dimension de la plaque d'implant.

36. Implant de disque intervertébral selon l'une des revendications précédentes,
**caractérisé en ce que** le noyau d'implant (19) est pourvu, au moins sur une face extérieure, d'un canal d'introduction (55), s'étendant depuis la bordure vers l'évidement (53), pour le tenon (51) de la plaque d'implant (15,17).

37. Implant de disque intervertébral selon l'une des revendications précédentes,
**caractérisé en ce qu'**au moins une plaque d'implant (15, 17) est pourvue, sur sa face intérieure, d'un canal d'introduction (57), s'étendant depuis la bordure vers le renfoncement (45), pour le noyau d'implant (19).

38. Procédé pour la fabrication d'un implant de disque intervertébral selon la revendication 1, dans lequel le coussin de soutien (277) est réalisé par injection, dans un processus d'injection de matière plastique, sur la coque (279, 281), en particulier sur les demi-coques, ou sur une couche intermédiaire (289, 291) agencée dans l'état fini entre le coussin de soutien (277) et la coque (279, 281).

39. Procédé selon la revendication 38,
**caractérisé en ce que**, pour réaliser un matériau composite entre le coussin de soutien (270) et la coque (279, 281), on choisit pour le coussin de soutien (277) à appliquer par injection un matériau qui présente une température de fusion plus élevée que le matériau de la coque (279, 281).

40. Procédé selon la revendication 38 ou 39,
**caractérisé en ce que** la couche intermédiaire (289, 291) est réalisée en métal.

41. Procédé selon l'une des revendications 38 à 40,
**caractérisé en ce que** lors de l'injection du coussin de soutien (277), on forme par injection des ressauts ou des contre-dépouilles (295) réalisé(e)s contre la face intérieure de la coque (279, 281) ou de la couche intermédiaire (289, 291).
